(19) Europäisches Patentamt / European Patent Office / Office européen des brevets

(11) EP 1 752 475 B1

(12) **EUROPEAN PATENT SPECIFICATION**

(45) Date of publication and mention of the grant of the patent:
**05.11.2008 Bulletin 2008/45**

(51) Int Cl.:
***C08F 8/16*** *(2006.01)* ***C08F 220/26*** *(2006.01)*
***G02B 1/04*** *(2006.01)* *C08F 8/48* *(2006.01)*

(21) Application number: **06016726.9**

(22) Date of filing: **10.08.2006**

(54) **Lactone ring-containing polymer having few foreign matters and not easily causing gelation, and its applications**

Lacton-Ring enthaltendes Polymer welches nicht leicht Vergelung unterliegt und wenig Fremdstoffe hat, und seine Anwendungen

Polymère contenant un cycle lactonique, contenant peu de substances étrangères et qui n'est pas aisément gélifié, ainsi que ses applications

(84) Designated Contracting States:
**DE FR**

(30) Priority: **11.08.2005 JP 2005233059**

(43) Date of publication of application:
**14.02.2007 Bulletin 2007/07**

(73) Proprietor: **NIPPON SHOKUBAI CO., LTD.**
**Osaka-shi, Osaka-fu 541 (JP)**

(72) Inventors:
- **Ueda, Ken-ichi**
  **Nara-shi**
  **Nara (JP)**
- **Otome, Shigeo**
  **Kita-ku**
  **Kyoto-shi (JP)**
- **Maeda, Nobuhiro**
  **Kobe-shi**
  **Hyogo (JP)**
- **Nakanishi, Hidetaka**
  **Higashiyodogawa-ku, Osaka-shi**
  **Osaka (JP)**
- **Izumi, Hiroko**
  **Tsukuba-shi**
  **Ibaraki (JP)**
- **Nagano, Hideaki**
  **Himeji-shi**
  **Hyogo (JP)**

(74) Representative: **Müller-Boré & Partner**
**Patentanwälte**
**Grafinger Strasse 2**
**81671 München (DE)**

(56) References cited:
**EP-A- 1 008 606** **JP-A- 9 067 310**

- **DATABASE WPI Week 199747 Derwent Publications Ltd., London, GB; AN 1997-508865 XP002408014 -& JP 09 241323 A (MITSUBISHI RAYON CO LTD) 16 September 1997 (1997-09-16)**
- **DATABASE WPI Week 200107 Derwent Publications Ltd., London, GB; AN 2001-053947 XP002408015 -& JP 2000 302815 A (TOA GOSEI CHEM IND LTD) 31 October 2000 (2000-10-31)**
- **DATABASE WPI Week 200447 Derwent Publications Ltd., London, GB; AN 2004-491751 XP002408017 -& JP 2004 168882 A (TORAY IND INC) 17 June 2004 (2004-06-17)**
- **DATABASE WPI Week 200202 Derwent Publications Ltd., London, GB; AN 2002-013222 XP002408018 -& JP 2001 151814 A (NIPPON SHOKUBAI CO LTD) 5 June 2001 (2001-06-05)**
- **DATABASE WPI Week 199818 Derwent Publications Ltd., London, GB; AN 1998-201624 XP002408020 -& JP 10 053547 A (NIPPON SHOKUBAI CO LTD) 24 February 1998 (1998-02-24)**
- **DATABASE WPI Week 200609 Derwent Publications Ltd., London, GB; AN 2006-083111 XP002408016 -& JP 2006 008901 A (KANEKA CORP) 12 January 2006 (2006-01-12)**
- **DATABASE WPI Week 200623 Derwent Publications Ltd., London, GB; AN 2006-222013 XP002408019 -& WO 2006/025445 A1 (NIPPON SHOKUBAI CO LTD) 9 March 2006 (2006-03-09)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 752 475 B1

- DATABASE WPI Week 200642 Derwent Publications Ltd., London, GB; AN 2006-406937 XP002408021 -& JP 2006 143635 A (SANYO CHEM IND LTD) 8 June 2006 (2006-06-08)

## Description

The present invention relates to a lactone ring-containing polymer having few foreign matters and not easily causing gelation, and its applications.

As resins having transparency, methacrylic resins have been known so far. Because methacrylic resins are excellent in transparency as well as in surface gloss and weather resistance, and are well balanced in mechanical strength, forming and processing properties, and surface hardness, the resins have widely been used in applications related to optics in automobiles, home electric appliances, and the like. However, because glass transition temperatures of methacrylic resins are about 110°C, it was difficult to used the resins in the fields where heat resistance is needed.

As transparent heat-resistant resins having transparency and heat resistance and, in addition, having various characteristics such as mechanical strength and forming and processing properties, recently, some lactone ring-containing polymers have been proposed, which are obtained by subjecting polymers having a hydroxy group and an ester group in the molecular chain to a lactone cyclocondensation reaction. For example, one of them is a polymer in which a lactone ring has been produced by the condensation of a hydroxy group and an ester group existing in the same molecule by subjecting 2-hydroxymethylacrylic acid alkyl ester / methyl methacrylate copolymer to the dealcoholization reaction, which is a kind of ester interchange.

Because in case of producing a lactone ring-containing polymer, after obtaining a polymer having a hydroxy group and an ester group in the molecular chain, the polymer passes through the lactone cyclocondensation process, when there is a branching in the polymer molecular chain, dealcoholization reaction occurs also between the molecules and "polymer gel" is easy to be generated.

Moreover, in the conventional producing method, for example, as described in the Example of the Japanese Patent Application Laid-Open (JP-A) No. 2000-230016, generally, although t-butyl type peroxides were used as a polymerization initiator, this kind of polymerization initiator has the high ability of withdrawing hydrogen atoms from a polymer molecular chain, so that the branching of the polymer molecular chain and the crosslinking between the molecules occurs and polymer gel is easy to be generated, too.

Further, in the Example of the Japanese Patent Application Laid-Open (JP-A) No. 2005-146084, although examples of experiments using t-amyl type peroxide and initiators of azo series are described, the weight average molecular weight of the obtained polymers is relatively high in the range of from 180,000 to 500, 000. In such molecular weight region, polymer gel is easy to be generated.

JP-A-09-241323 describes a thermal resistant polymer which is used for molding materials or optical materials and which is produced by heating a polymer having monomer units of a specific formula at 150°C or more, wherein the solvent may contain esterifying catalyst(s) or ester-interchange catalysts. EP-A-1 008 606 describes a production process for a transparent heat-resistant resin, which comprises the step of running a dealcoholation reaction of a polymer having a hydroxyl group and an ester group in its molecular chain to introduce a lactone ring structure into the polymer to obtain a transparent resin having the heat resistance, and is characterized in that the dealcoholation reaction is run in the presence of a solvent, and further characterized by further comprising a devolatization step which is carried out jointly with the dealcoholation reaction. JP-A-2000-302815 describes a manufacturing method of a heat-resistant resin comprising the step of dealcoholation of a homogeneous polymer of a specific poly-alpha-hydroxymethylacrylate in the presence of a quaternary ammonium salt. JP-A-2004-168882 describes a copolymer containing 5 wt.-% or more of a specific lactone ring-system unit. JP-A-2001-151814 describes a method of producing a transparent heat-resistant resin comprising the step of introducing a lactone ring structure into a source polymer which has hydroxyl groups and ester groups in its polymer chain by a dealcoholation reaction using an organic phosphorus catalyst. JP-A-10-053547 describes a method for purification of vinyl compounds containing hydroxyl groups, the method comprising the step of washing a specific vinyl compound containing hydroxyl groups with an aqueous solution of at least one compound selected from sulfites, amines and salts, and then separating the oil layer and the aqueous layer. JP-A-09067310 describes heat-treating a crude $\alpha$-hydroxyalkyl acrylate containing an acetal compound and purifying it.

Well, for example, in the optical applications such as optical film, it is a problem that foreign matters exist in an optical material, and so the foreign matters are necessary to be reduced as much as possible. Although lactone ring-containing polymers obtained by the conventional producing method are forming materials having excellent optical characteristics, there was a problem that gels were generated by heating the material when forming it, in addition that polymer gels exist also in the polymer pellets as foreign matters. Thus, particularly in optical applications, such a forming material was required to have few foreign matters at the stage of polymer pellets and not easily to cause gelation even by being heated when forming the polymer.

Under these circumstances, the problem to be solved by the present invention is to provide a lactone ring-containing polymer having few foreign matters and not easily causing gelation, in addition to being excellent in transparency and heat resistance as well as having the desired properties such as mechanical strength and forming and processing properties.

As a result of various studies, the present inventors have found that when producing the lactone ring-containing

polymer, a lactone ring-containing polymer, in which the generation of the polymer gel can be controlled and in case of being used as a forming material, the polymer does not easily cause gelation to increase the amount of polymer gels even if the polymer is heated, for example, being subjected to retention under heating during the extrusion forming, can be obtained by using a polymerization initiator having the low ability of withdrawing hydrogen atoms from a polymer molecular chain such as a t-amyl type peroxide, an initiator of azo series, or an initiator of living radical series, instead of a t-butyl type peroxide, and by limiting the molecular weight in the prescribed range, and also have found that the generation of the polymer gel can very effectively be controlled in the lactone ring-containing polymer by using a 2-hydroxymethylacrylic acid ester purified with the specific method as a monomer and using a t-amyl type peroxide as a polymerization initiator when producing the lactone ring-containing polymer, thereby completing the present invention.

That is, the present invention provides a lactone ring-containing polymer meeting a condition:

(1) a content of polymer gels of 50 μm or greater in average particle diameter is smaller than or equal to 100 pieces/100 g, and the weight average molecular weight of the polymer is 50,000 to 170,000; and in a preferred embodiment (2) the increasing rate of viscosity after being heated at 280°C for 30 minutes is 2.0 times or smaller.

The lactone ring-containing polymer of the present invention preferably has a lactone ring structure of formula (1):

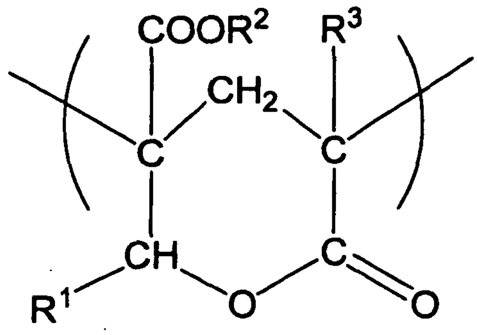

(1)

wherein $R^1$, $R^2$, and $R^3$ each independently means a hydrogen atom or an organic residue having 1 to 20 carbon atoms, in which the organic residue may contain an oxygen atom(s).

The present invention further provides a forming material producible by heating and granulating the above lactone ring-containing polymer, and an optical component formed from the forming material. The optical component may include a light guide material, an optical lens, and an optical film.

In the course of the present invention, there is provided, as a monomer suitable for producing the above lactone ring-containing polymer using a t-amyl type peroxide as a polymerization initiator, a 2-hydroxymethylacrylic acid ester of formula (2):

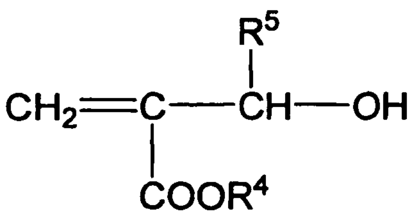

(2)

wherein $R^4$ and $R^5$ each independently means a hydrogen atom or an organic residue having 1 to 20 carbon atoms, in which the organic residue may contain an oxygen atom(s), turbidity of the ester after heating test at 90°C for two hours being 0.05 or lower.

According to the lactone ring-containing polymer of the present invention, because a specific polymerization initiator such as a t-amyl type peroxide is used and the molecular weight is limited in the prescribed range, for example, very excellent optical components can be provided, particularly having very few foreign matters (polymer gels) and not easily causing gelation even by being heated when forming, in addition to being excellent in transparency and heat resistance as well as having the desired properties such as mechanical strength and forming and processing properties.

<<Lactone ring-containing polymer>>

The lactone ring-containing polymer of the present invention meets, in a certain aspect, the conditions that the content of polymer gels of 50 μm or greater in average particle diameter is smaller than or equal to 100 pieces/100 g and the weight average molecular weight of the polymer is 50,000 to 170,000. The term "polymer gel" as used herein means the one that a polymer has constituted a three-dimensional network structure by chemical bonding or by interaction between polymer molecular chains. Further, in the present invention, the terms "polymer gel" and "foreign matter" are used exchangeably with each other.

When a lactone ring-containing polymer is used in optical applications as a forming material, a polymer gel of greater than 50 μm in average particle diameter becomes a problem. Thus, in the present invention, the content of polymer gels of 50 μm or greater in average particle diameter is defined to be smaller than or equal to 100 pieces/100 g. Further, the content of polymer gels should be determined as follows: after dissolving 100 g of a lactone ring-containing polymer in 500 ml of a solvent (for example, methyl ethyl ketone or the like), in which the lactone ring-containing polymer is soluble and which has been purified by microfiltration, the solution is filtered with a membrane filter made of polytetrafluoroethylene, having an average pore size of 1.0 μm, and then the number of pieces of polymer gels of 50 μm or greater in average particle diameter which are remained on the filter is counted by visual observation under a microscope. When the content of polymer gels is greater than 100 pieces/100 g, in case of being used as a forming material, the polymer may be unsuitable for optical applications. The content of polymer gels may preferably be smaller than or equal to 50 pieces/100 g, more preferably smaller than or equal to 20 pieces/100 g. The lower limit of the content of polymer gels is ideally 0 pieces/100 g.

Moreover, the lactone ring-containing polymer has a tendency to generate polymer gels easily when the molecular weight of the polymer is increased. Thus, in the present invention, the weight average molecular weight of the polymer is defined to be 50,000 to 170,000. The weight average molecular weight may preferably be in the range of from 100,000 to 150,000. Further, the weight average molecular weight of the polymer is assumed to be a value determined by polystyrene calibration using the gel permeation chromatography. When the weight average molecular weight of the polymer is smaller than 50, 000, the melt flow rate of the polymer may become high, and in case of being used as a forming material, the forming and processing of the polymer, for example, by the melt extrusion process and the like may become difficult. In contrast, when the weight average molecular weight of the polymer is greater than 170,000, the melt flow rate of the polymer may become low as well as the polymer gel may easily be generated, and in case of being used as a forming material, the forming and processing of the polymer, for example, by the injection forming process and the like may become difficult.

The lactone ring-containing polymer of the present invention meets, in a further preferred aspect, the condition that the increasing rate of viscosity after being heated at 280°C for 30 minutes is 2.0 times or smaller. The term "the increasing rate of viscosity" as used herein means the ratio of melt viscosities (after heating/before heating) obtained as follows: the melt viscosities of the polymer before and after being heated at 280°C for 30 minutes are measured in the conditions that the temperature is 250°C, the load is 10 $kgf/cm^2$, and the die shape is 0.5 mmφ × 1 mm. When the increasing rate of viscosity of the polymer is greater than 2.0 times, in case of being used as a forming material, the forming and processing properties of the polymer may be lowered. Although the low limit of the increasing rate of viscosity of the polymer is ideally 1.0 time; however, it is 0.8 times, considering the case where a part of the polymer may be decomposed. In addition, the increasing rate of viscosity is an index of gelation which is generated when a polymer is heated, and the polymer with its small value does not easily cause gelation.

As for the lactone ring-containing polymer of the present invention, the mass decreasing rate in the range of from 150°C to 300°C in the dynamic TG measurement may preferably be 1% or smaller, more preferably 0.5% or smaller, and still more preferably 0.3% or smaller.

Because the lactone ring-containing polymer of the present invention has a high cyclocondensation reaction rate, faults that bubbles and silver streaks enter in the formed goods after forming can be avoided. In addition, because the lactone ring structure is introduced enough into the polymer by the high cyclocondensation reaction rate, the obtained lactone ring-containing polymer has very high heat resistance.

When the 15% by mass chloroform solution of the lactone ring-containing polymer of the present invention is prepared, the yellow index (YI) of the solution may preferably be 6 or smaller, more preferably 3 or smaller, still more preferably 2 or smaller, and particularly preferably 1 or smaller. When the yellow index (YI) is greater than 6, the transparency is ruined by coloration, and the polymer may be unable to be used particularly for optical applications.

As for the lactone ring-containing polymer of the present invention, 5% mass decreasing temperature in the thermogravimetric analysis (TG) may preferably be 300°C or higher, more preferably 320°C or higher, and still more preferably 330°C or higher. The 5% mass decreasing temperature in the thermogravimetric analysis (TG) is an index of thermal stability, and when this value is lower than 300°C, enough thermal stability may be unable to be exhibited.

As for the lactone ring-containing polymer of the present invention, the glass transition temperature (Tg) may preferably be 100°C or higher, more preferably 110°C or higher, still more preferably 120°C or higher, and particularly

preferably 130°C or higher.

As for the lactone ring-containing polymer of the present invention, the total amount of residual volatile components contained may preferably be 1,500 ppm or smaller, and more preferably 1, 000 ppm or smaller. When the total amount of residual volatile components is greater than 1,500 ppm, changing in properties when forming the polymer may cause a defective forming with coloring, foaming, silver streaks, or the like.

As for the lactone ring-containing polymer of the present invention, all light transmittance measured by the method in accordance with ASTM-D-1003 on formed goods obtained by injection forming may preferably be 85% or higher, more preferably 88% or higher, and still more preferably 90% or higher. All light transmittance is an index of transparency, and when this value is lower than 85%, the transparency is decreased, and the polymer may be unable to be used particularly for optical applications.

The lactone ring-containing polymer of the present invention preferably has a lactone ring structure of formula (1):

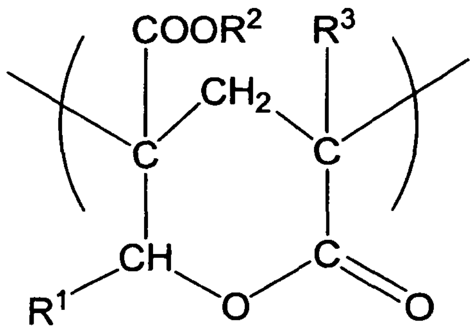

**(1)**

wherein $R^1$, $R^2$, and $R^3$ each independently means a hydrogen atom or an organic residue having 1 to 20 carbon atoms, in which the organic residue may contain an oxygen atom(s).

The proportion of the lactone ring structure of the above formula (1) contained in the structure of the lactone ring-containing polymer may preferably be 5% to 90% by mass, more preferably 10% to 70% by mass, still more preferably 10% to 60% by mass, and particularly preferably 10% to 50% by mass. When the proportion of the lactone ring structure contained is smaller than 5% by mass, the heat resistance, solvent resistance, and surface hardness of the obtained polymer may be decreased. In contrast, when the proportion of the lactone ring structure contained is greater than 90% by mass, the forming and processing properties of the obtained polymer may be decreased.

The lactone ring-containing polymer may have any structure other than the lactone ring structure of the above formula (1). The structure other than the lactone ring structure of the above formula (1) is not particularly limited, but, for example, as described later as the producing method of the lactone ring-containing polymer, such a polymer structural unit (repeated structural unit) may be preferable formed by polymerizing at least one kind of monomer selected from the group consisting of (meth)acrylates, hydroxy group-containing monomers, and monomers of formula (3):

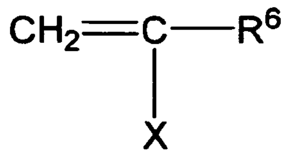

**(3)**

wherein $R^6$ means a hydrogen atom or a methyl group, X means a hydrogen atom, an alkyl group having 1 to 20 carbon atoms, an aryl group, an -OAc group, a -CN group, a -CO-$R^7$ group, or a -COOH group, Ac means an acetyl group, and $R^7$ means a hydrogen atom or an organic residue having 1 to 20 carbon atoms.

Although the producing method of the lactone ring-containing polymer is not particularly limited, for example, the polymer is obtained as follows: after polymer (a) having a hydroxy group and an ester group in the molecular chain is obtained by the polymerization process, the lactone cyclocondensation process for introducing the lactone ring structure into the polymer is carried out by heat treating the obtained polymer (a).

In the polymerization process, a polymer having a hydroxy group and an ester group in the molecular chain is obtained by carrying out the polymerization reaction of the monomer components in which, for example, a monomer of formula (2):

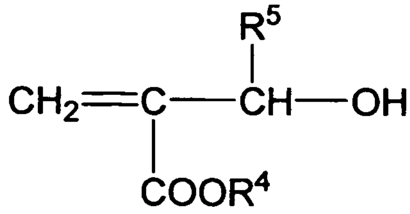

(2)

wherein $R^4$ and $R^5$ each independently means a hydrogen atom or an organic residue having 1 to 20 carbon atoms, in which the organic residue may contain an oxygen atom(s), (hereinafter referred to as "2-hydroxymethylacrylic acid ester"), is contained.

As the monomers of the above formula (2), for example, (a) monomers in which the substituent group shown by $R^4$ is a hydrogen atom may include 2-hydroxymethylacrylic acid, 2-(1-hydroxyethyl)acrylic acid, 2-(1-hydroxybutyl) acrylic acid, and. 2-(1-hydroxy-2-ethylhexyl)acrylic acid; (b) monomers in which the substituent group shown by $R^4$ is an alkyl group having 1 to 18 carbon atoms may include methyl 2-hydroxymethylacrylate, methyl 2-(1-hydroxyethyl) acrylate, methyl 2-(1-hydroxybutyl)acrylate, methyl 2-(1-hydroxy-2-ethylhexyl)acrylate, ethyl 2-hydroxymethylacrylate, ethyl 2-(1-hydroxyethyl)acrylate, ethyl 2-(1-hydroxybutyl)acrylate, ethyl 2-(1-hydroxy-2-ethylhexyl)acrylate, n-propyl 2-hydroxymethylacrylate, n-propyl 2-(1-hydroxyethyl)acrylate, n-propyl 2-(1-hydroxybutyl)acrylate, n-propyl 2-(1-hydroxy-2-ethylhexyl)acrylate, isopropyl 2-hydroxymethylacrylate, isopropyl 2-(1-hydroxyethyl)acrylate, isopropyl 2-(1-hydroxybutyl)acrylate, isopropyl 2-(1-hydroxy-2-ethyhexyl)acrylate, n-butyl 2-hydroxymethylacrylate, isobutyl 2-hydroxymethylacrylate, t-butyl 2-hydroxymethylacrylate, n-octyl 2-hydroxymethylacrylate, isooctyl 2-hydroxymethylacrylate, 2-ethylhexyl 2-hydroxymethylacrylate, and stearyl 2-hydroxymethylacrylate; (c) monomers in which the substituent group shown by $R^4$ is a cycloalkyl group having 3 to 10 carbon atoms may include cyclopentyl 2-hydroxymethylacrylate, cyclopentyl 2-(1-hydroxyethyl)acrylate, cyclopentyl 2-(1-hydroxybutyl)acrylate, cyclopentyl 2-(1-hydroxy-2-ethylhexyl)acrylate, and cyclohexyl 2-hydroxymethylacrylate; (d) monomers in which the substituent group shown by $R^4$ is an aryl group may include phenyl 2-hydroxymethylacrylate, phenyl 2-(1-hydroxyethyl)acrylate, phenyl 2-(1-hydroxybutyl)acrylate, phenyl 2-(1-hydroxy-2-ethylhexyl)acrylate, o-methoxyphenyl 2-hydroxymethylacrylate, p-methoxyphenyl 2-hydroxymethylacrylate, p-nitrophenyl 2-hydroxymethylacrylate, o-methylphenyl 2-hydroxymethylacrylate, p-methylphenyl 2-hydroxymethylacrylate, and p-t-butylphenyl 2-hydroxymethylacrylate. These monomers may be used alone, or two or more kinds of them may also be used in combination. In these monomers, methyl 2-hydroxymethylacrylate, ethyl 2-hydroxymethylacrylate, n-butyl 2-hydroxymethylacrylate, 2-ethylhexyl 2-hydroxymethylacrylate, 2-hydroxyethyl 2-hydroxymethylacrylate, and 2-hydroxypropyl 2-hydroxymethylacrylate are suitable, and methyl 2-hydroxymethylacrylate is particularly suitable because of its high effect of improving heat resistance.

The proportion of the monomer of the above formula (2) contained in the monomer components to be subjected to a polymerization process may preferably be 2.5% to 50% by mass, more preferably 5% to 40% by mass, still more preferably 5% to 35% by mass, and particularly preferably 5% to 30% by mass. When the proportion of the monomer of the above formula (2) contained is smaller than 2.5% by mass, the heat resistance, solvent resistance, and surface hardness of the obtained polymer may be decreased. In contrast, when the proportion of the monomer of the above formula (2) contained is greater than 50% by mass, gelation may be caused in the polymerization process and the lactone cyclocondensation process, and the forming and processing properties of the obtained polymer may be decreased.

Any monomer other than the monomer of the above formula (2) may be combined in the monomer components to be subjected to a polymerization process. Such monomers are not particularly limited and may include (meth) acrylic acid esters, and monomers of formula (3) :

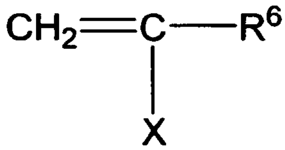

(3)

wherein $R^6$ means a hydrogen atom or a methyl group, X means a hydrogen atom, and an alkyl group having 1 to 20 carbon atoms, an aryl group, an -OAc group, a -CN group, a -CO-$R^7$ group, or a -COOH group, Ac means an acetyl group, and $R^7$ means a hydrogen atom or an organic residue having 1 to 20 carbon atoms. These monomers may be used alone, or two or more kinds of them may also be used in combination.

The (meth) acrylic acid esters are not particularly limited, so long as they are (meth) acrylic acid esters other

than monomers of the above formula (2), and may include acrylic acid esters such as methyl acrylate, ethyl acrylate, n-butyl acrylate, isobutyl acrylate, t-butyl acrylate, cyclohexyl acrylate, and benzyl acrylate; and methacrylic acid esters such as methyl methacrylate, ethyl methacrylate, propyl methacrylate, n-butyl methacrylate, isobutyl methacrylate, t-butyl methacrylate, cyclohexyl methacrylate, and benzyl methacrylate. These (meth)acrylic acid esters may be used alone, or two or more kinds of them may also be used in combination. In these (meth)acrylic acids, methyl methacrylate may be particularly preferable because the obtained polymer is excellent in heat resistance and transparency.

When (meth) acrylic acid esters other than monomers of the above formula (2) are used, the proportion of the (meth) acrylic acid esters contained in the monomer components to be subjected to a polymerization process may preferably be 10% to 97.5% by mass, more preferably 10% to 95% by mass, still more preferably 40% to 95% by mass, and particularly preferably 50% to 95% by mass, for the purpose of exhibiting the effect of the present invention enough.

The monomers of the above formula (3) may include styrene, $\alpha$-methyl styrene, vinyl toluene, acrylonitrile, methyl vinyl ketone, ethylene, propylene, and vinyl acetate. These monomers may be used alone, or two or more kinds of them may also be used in combination.

When the monomers of the above formula (3) are used, the proportion of the monomer contained in the monomer components to be subjected to a polymerization process may preferably be 0% to 30% by mass, more preferably 0% to 20% by mass, still more preferably 0% to 15% by mass, and particularly preferably to be 0% to 10% by mass, for the purpose of exhibiting the effect of the present invention enough.

As the form of the polymerization reaction to obtain a polymer having a hydroxy group and an ester group in the molecular chain by polymerizing monomer components, a polymerization form of using a solvent may be preferable, and the solution polymerization may be particularly preferable. Moreover, because the living radical polymerization consists only of the initiation reaction and the propagation reaction and no side reaction of deactivating the propagation site such as termination and chain transfer occurs, the living radical polymerization does not pull out few hydrogen atoms from the polymer molecular chain and may be particularly suitable to control the generation of polymer gels.

Although the polymerization temperature and the polymerization time may vary according to the kinds and the proportions of monomers used, it may be preferable, for example, that the polymerization temperature is 0°C to 150°C and the polymerization time is 0.5 to 20 hours, and more preferable that the polymerization temperature is 80°C to 140°C and the polymerization time is 1 to 10 hours.

In case of the polymerization form of using a solvent, the polymerization solvents are not particularly limited, but may include aromatic hydrocarbon solvents such as toluene, xylene, and ethylbenzene; ketone solvents such as methyl ethyl ketone and methyl isobutyl ketone; ether solvents such as tetrahydrofuran. These solvents may be used alone, or two or more kinds of them may also be used in combination. Moreover, when the boiling point of the solvent is too high, because the remaining volatile matters in the lactone ring-containing polymer finally obtained increase, a solvent having a boiling point of 50°C to 200°C may be preferable.

During the polymerization reaction, a polymerization initiator may be added, if necessary. The polymerization initiator is not particularly limited, so long as it has the low ability of withdrawing a hydrogen atom from a polymer molecular chain, but may include t-amyl type peroxides such as t-amyl peroxy-2-ethylhexanoate, t-amyl peroxyisononanoate, t-amyl peroxyacetate, t-amyl peroxybenzoate, and 1,1-di(t-amylperoxy)cyclohexane; initiators of azo series, such as 2,2'-azobis(isobutyronitrile), 1,1'-azobis(cyclohexane-1-carbonitrile), 2,2'-azobis(2,4-dimethylvaleronitrile), and dimethyl 2,2'-azobisisobutylate; and initiators of living radical series, such as dichlorotris(triphenylphosphine) ruthenium, aluminum triisopropoxide, and 2,2'-dichloroacetophenone. These polymerization initiators may be used alone, or two or more kinds of them may also be used in combination. In these initiators, as described later, when the monomers of the above formula (2) are purified by the specific method, because the generation of the polymer gel in the lactone ring-containing polymer can very effectively be controlled, t-amyl type peroxides may be particularly suitable. Further, the amount of the polymerization initiator used has only to be set properly according to the combination of monomers and the reaction conditions, and is not particularly limited.

When polymerization is carried out, the concentration of the polymer produced in the polymerization reaction mixture may preferably be controlled to be 50% by mass or lower in order to suppress the gelation of the reaction liquid. Specifically, when the concentration of the polymer produced in the polymerization reaction mixture is greater than 50% by mass, it may be preferable to add the polymerization solvent properly in the polymerization reaction mixture so that the concentration is controlled to be 50% by mass or less. The concentration of the polymer produced in the polymerization reaction mixture may more preferably be 45% by mass or lower, and still more preferably 40% by mass or lower. Further, the concentration of the polymer produced in the polymerization reaction mixture is too low, so that the productivity is decreased. The concentration of the polymer produced in the polymerization reaction mixture may preferably be 10% by mass or higher, and more preferably 20% by mass or higher.

The form of properly adding the polymerization solvent in the polymerization reaction mixture is not particularly limited, for example, the polymerization solvent may be added continuously, and may be added intermittently. Through controlling the concentration of the polymer produced in the polymerization reaction mixture as described above, the gelation of the reaction liquid can be suppressed enough. In particular, even when the proportion of the hydroxy group

and the ester group in the molecular chain is increased in order to increase the proportion of the lactone ring-containing and consequently improve the heat resistance of the polymer, the gelation of the reaction liquid can be suppressed enough. Although the polymerization solvent to be added may be the same solvent as that used at the time of the initial feeding for the polymerization reaction, and may be the different kind of solvent, it may be preferable to use the same solvent as that used at the time of the initial feeding for the polymerization reaction. Moreover, the polymerization solvent to be added may be a single solvent, and may be a mixture of two or more kinds of solvents.

In the polymerization reaction mixture obtained at the end of the polymerization process described above, although the solvent is generally contained other than the obtained polymer, it is not needed to remove the solvent completely and to take out the polymer in the state of the solid, and the polymer with the solvent contained may preferably be introduced into the next lactone cyclocondensation process. Moreover, if needed, after the polymer is taken out in the state of the solid, the suitable solvent may be added again in the next lactone cyclocondensation process.

The polymer obtained in the polymerization process is the polymer (a) having a hydroxy group and an ester group in the molecular chain, and the weight average molecular weight of the polymer (a) may preferably be 50,000 to 170,000, more preferably 60,000 to 170,000, and still more preferably 70,000 to 170,000. The polymer (a) obtained in the polymerization process is heat treated in the next lactone cyclocondensation process, and the lactone ring structure is introduced into the polymer, resulting in a lactone ring-containing polymer.

The reaction of introducing the lactone ring structure into the polymer (a) is the reaction that the cyclocondensation of the hydroxy group and the ester group existing in the molecular chain of the polymer (a) are caused by heating the polymer (a) and the lactone ring structure is consequently produced, and alcohol is generated as a by-product in the cyclocondensation. High heat resistance is given by forming the lactone ring structure in the molecular chain of the polymer (in the main skeleton of the polymer). When reaction rate in the cyclocondensation reaction of introducing the lactone ring structure is insufficient, such situations may be caused that the heat resistance is not improved enough, and that the condensation reaction occurs while forming the polymer by the heat treatment and generated alcohol exists as bubbles and silver streaks in the formed goods.

The lactone ring-containing polymer obtained in the lactone cyclocondensation process may be preferable to have the lactone ring structure of formula (1):

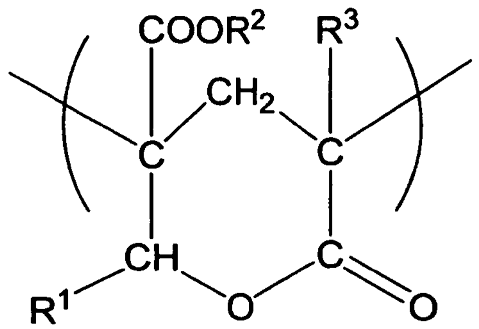

(1)

wherein $R^1$, $R^2$, and $R^3$ each independently means a hydrogen atom or an organic residue having 1 to 20 carbon atoms, in which the organic residue may contain an oxygen atom(s).

The method for heat treating the polymer (a) is not particularly limited, but any of the conventionally known methods has only to be used. For example, the polymerization reaction mixture obtained by the polymerization process including the solvent may be heat treated as it is. Alternatively, the polymer may be heat treated using a ring closure catalyst in the presence of a solvent, if necessary. Alternatively, heat treatment can be carried out with the use of any of the apparatuses such as a heating furnace and a reactor that are equipped with a vacuum device or a devolatilizing device to remove volatile components, and an extruder equipped with a devolatilizing device.

When the cyclocondensation reaction is carried out, in addition to the polymer (a), other thermoplastic resin may be allowed to coexist. Moreover, when the cyclocondensation reaction is carried out, if necessary, an esterification catalyst or a transesterification catalyst such as p-toluenesulfonic acid, which is generally used as a catalyst of the cyclocondensation reaction, may be used, or an organocarboxylic acid such as acetic acid, propionic acid, benzoic acid, acrylic acid, or methacrylic acid may be used as a catalyst. Further, as disclosed in Japanese Patent Application Laid-open (JP-A) Nos. 61-254608 and 61-261303, basic compounds, organocarboxylates, carbonates, and the like may be used.

Alternatively, as a catalyst of the cyclocondensation reaction, an organophosphorous compound may be used. Usable organophosphorous compounds may include alkyl(aryl)phosphonous acid and their monoesters and diesters, such as methylphosphonous acid, ethylphosphonous acid, and phenylphosphonous acid (however, these may be alkyl(aryl)phosphonic acids as tautomers); dialkyl (aryl) phosphonic acid and their esters, such as dimethylphosphonic acid, diethylphosphonic acid, diphenylphosphonic acid, phenylmethylphosphonic acid, and phenylethylphosphonic acid; alkyl (aryl)phosphonic acids and their monoesters and diesters, such as methylphosphonic acid, ethylphosphnonic acid, trifluoromethylphosphonic acid, and phenylphosphonic acid; alkyl(aryl)phosphinous acids and their esters, such as methylphosphinous acid, ethylphosphinous acid, and phenylphosphinous acid; phosphorous monoesters, diesters and triesters, such as methylphosphite, ethylphosphite, phenylphosphite, dimethylphosphite, diethylphosphite, diphenylphosphite, trimethylphosphite, triethylphosphite, and triphenylphosphite; phosphoric monoesters, diesters and triesters, such as methyl phosphate, ethyl phosphate, 2-ethylhexyl phosphate, octyl phosphate, isodecyl phosphate, lauryl phosphate, stearyl phosphate, isostearyl phosphate, phenyl phosphate, dimethyl phosphate, diethyl phosphate, di-2-ethylhexyl phosphate, diisodecyl phosphate, dilauryl phosphate, distearyl phosphate, diisostearyl phosphate, diphenyl phosphate, trimethyl phosphate, triethyl phosphate, triisodecyl phosphate, trilauryl phosphate, tristearyl phosphate, triisostearyl phosphate, and triphenyl phosphate; mono-, di-, or tri-alkyl(aryl)phosphines such as methylphosphine, ethylphosphine, phenylphosphine, dimethylphosphine, diethylphosphine, diphenylphosphine, trimethylphosphine, triethylphosphine, and triphenylphosphine; alkyl(aryl) halogen phosphines such as methyldichlorophosphine, ethyldichlorophosphine, phenyldichlorophosphine, dimethylchlorophosphine, diethylchlorophosphine, and diphenylchlorophosphine; mono-, di-, or tri-alkyl(aryl)phosphine oxides such as methylphosphine oxide, ethylphosphine oxide, phenylphosphine oxide, dimethylphosphine oxide, diethylphosphine oxide, diphenylphosphine oxide, trimethylphosphine oxide, triethylphosphine oxide, and triphenylphosphine oxide; halogenated tetraalkyl(aryl)phosphoniums such as tetramethylphosphonium chloride, tetraethylphosphonium chloride, and tetraphenylphosphonium chloride. These organic phosphorous compounds may be used alone, or two or more kinds of them may also be used in combination. In these organic phosphorous compounds, because catalytic activity is high and coloring is low, alkyl(aryl)phosphonous acids, phosphorous monoesters or diesters, phosphoric monoesters or diesters, and alkyl(aryl)phosphonic acids may be preferable, and alkyl(aryl)phosphonous acids, phosphorous monoesters or diesters, phosphoric monoesters or diesters may be more preferable, and alkyl(aryl) phosphonous acids and phosphoric monoesters or diesters may be particularly preferable.

Although the amount of the catalyst used in the cyclocondensation reaction is not particularly limited, for example, the amount may preferably be 0.001% to 5% by mass to the polymer (a), more preferably 0.01% to 2.5% by mass, still more preferably 0.01% to 1% by mass, and particularly preferably 0.05% to 0.5% by mass. When the amount of the catalyst used is smaller than 0.001% by mass, the reaction rate in the cyclocondensation reaction may be unable to be improved enough. In contrast, when the amount of the catalyst used is greater than 5% by mass, such problems may be caused that the obtained polymer is colored, or the polymer is cross-linked and its melt forming becomes difficult.

The addition timing of the catalyst is not particularly limited, but for example, the catalyst may be added at the initial stage of the reaction, may be added while reacting, and may be added by both timing.

The cyclocondensation reaction may preferably be carried out in the presence of a solvent, and the devolatilizing process may preferably be used together at the time of the cyclocondensation reaction. In this case, two patterns can be exemplified, that is, the one that the devolatilizing process is used together throughout the cyclocondensation reaction, and the other that the devolatilizing process is used together not throughout the cyclocondensation reaction but only in the part of the reaction. In the method in which the devolatilizing process is used together, alcohol produced as a by-product in the cyclocondensation reaction is compulsorily volatilized and removed, and consequently the equilibrium of the reaction becomes advantageous to the generation side.

The devolatilizing process means the process where the volatile matters including the solvent and the remaining monomers, and the alcohol as a by-product produced by the cyclocondensation reaction leading to the lactone ring structure are removed under reduced pressure and heating conditions, if necessary. When this removing treatment is insufficient, the remaining volatile matters in the obtained polymer increase, and defective forming such as coloring by changing in quality at the time of forming, and generation of bubbles and silver streaks may occur.

In case of the pattern of using the devolatilizing process together throughout the cyclocondensation reaction, although the devices used are not particularly limited, for example, in order to carry out the present invention more effectively, it may be preferable to use a devolatilizing device comprising a heat exchanger and a devolatilizing vessel, a vented extruder, and an apparatus in which a devolatilizing device and an extruder are arranged in series. It may be more preferable to use a devolatilizing device comprising a heat exchanger and a devolatilizing vessel, or a vented extruder.

The reaction temperature in case of using a devolatilizing device comprising of a heat exchanger and a devolatilizing vessel may preferably be 150°C to 350°C, and more preferably 200°C to 300°C. When the reaction temperature is lower than 150°C, it may occur that the cyclocondensation reaction becomes insufficient and remaining volatile matters are increased. In contrast, when the reaction temperature is higher than 350°C, the obtained polymer may be colored or decomposed.

The reaction pressure in case of using a devolatilizing device comprising a heat exchanger and a devolatilizing vessel may preferably be 931 to 1.33 hPa (700 to 1 mmHg), and more preferably 798 to 66.5 hPa (600 to 50 mmHg). When the reaction pressure is higher than 931 hPa (700 mmHg), volatile matters including alcohol may remain easily.

In contrast, when the reaction pressure is lower than 1.33 hPa (1 mmHg), it may be difficult to carry out the process on an industrial scale.

When a vented extruder is used, the number of vents may be acceptable in either one piece or two or more pieces, but the extruder having two or more vents may be preferable.

The reaction temperature in case of using a vented extruder may preferably be 150°C to 350°C, and more preferably 200°C to 300°C. When the reaction temperature is lower than 150°C, it may occur that the cyclocondensation reaction becomes insufficient and remaining volatile matters are increased. In contrast, when the reaction temperature is higher than 350°C, the obtained polymer may be colored or decomposed.

The reaction pressure in case of using a vented extruder may preferably be 931 to 1.33 hPa (700 to 1 mmHg), and more preferably 798 to 13.3 hPa (600 to 10 mmHg). When the reaction pressure is greater than 931 hPa (700 mmHg), volatile matters including alcohol may remain easily. In contrast, when the reaction pressure is lower than 1.33 hPa (1 mmHg), it might be difficult to carry out the process on an industrial scale.

Further, in case of the pattern of using the devolatilizing process together throughout the cyclocondensation reaction, as described later, because the physical properties of the lactone ring-containing polymer obtained may be deteriorated in the severe heat treating condition, it may be preferable to carry out the devolatilizing process with a vented extruder using the above dealcoholization catalyst under a condition as mild as possible.

Moreover, in case of the pattern of using the devolatilizing process together throughout the cyclocondensation reaction, although the polymer (a) obtained in the polymerization process may preferably be introduced together with a solvent into a cyclocondensation reaction device, in which case the polymer (a) may be passed through a cyclocondensation reaction device such as a vented extruder once more, if necessary.

The devolatilizing process may be carried out in such a pattern as to be used together not throughout the cyclocondensation reaction but only in the part of the reaction. For example, the reaction pattern is carried out as follows: the cyclocondensation reaction is allowed to progress beforehand to some degree by further heating the device in which the polymer (a) has been produced, and using partly the devolatilizing process together, if necessary, and after that the cyclocondensation reaction is carried out using the devolatilizing process at the same time and thus the reaction is completed.

In case of the pattern of using the devolatilizing process together throughout the cyclocondensation reaction as described above, for example, when the polymer (a) is heat treated using a twin screw extruder at a high temperature of about 250°C or higher, the physical properties of the lactone ring-containing polymer obtained may be deteriorated because the polymer is partly decomposed owing to the difference in the thermal history before the cyclocondensation reaction. Accordingly, before the cyclocondensation reaction is carried out using the devolatilizing process together at the same time, if the cyclocondensation reaction is allowed to progress beforehand to some degree, the reaction condition in the latter stage can be made mild and consequently the deterioration of the physical properties of the lactone ring-containing polymer obtained can preferably be controlled. As the particularly preferable pattern, for example, the pattern of starting the devolatilizing process at a time interval after the start of the cyclocondensation reaction, that is, such a pattern is exemplified that the reaction rate in the cyclocondensation reaction is raised beforehand to some degree by condensing and cyclizing a hydroxy group and an ester group existing in the molecular chain of the polymer (a) obtained in the polymerization process, and then the cyclocondensation reaction is carried out using the devolatilizing process together at the same time. Specifically, the following pattern may be preferably exemplified: the cyclocondensation reaction is allowed to progress beforehand to some degree of the reaction rate using a kettle type reactor in the presence of a solvent, and then the cyclocondensation reaction is completed with the reactor equipped with a devolatilizing device, for example, a devolatilizing device comprising a heat exchanger and a devolatilizing vessel, a vented extruder, or the like. In particular, in case of this pattern, it may be more preferable that a catalyst exists for the cyclocondensation reaction.

As described above, the method that the reaction rate in the cyclocondensation reaction is raised beforehand to some degree by condensing and cyclizing the hydroxy group and the ester group existing in the molecular chain of the polymer (a) obtained in the polymerization process, and then the cyclocondensation reaction is carried out using the devolatilizing process together at the same time may be a preferable pattern for obtaining the lactone ring-containing polymer in the present invention. In this pattern, the lactone ring-containing polymer, in which the glass transition temperature is higher, the reaction rate in the cyclocondensation reaction is raised higher, and the heat resistance is excellent, can be obtained. In this case, as a criterion for the reaction rate in the cyclocondensation reaction, the mass decreasing rate in the range of from 150°C to 300°C in the dynamic TG measurement shown in Examples may preferably be 2% or lower, more preferably 1.5% or lower, and still more preferably 1% or lower.

The reactor, which can be adopted when the cyclocondensation reaction is carried out in advance before the cyclocondensation reaction using the devolatilizing process together at the same time, is not particularly limited, and includes, for example, an autoclave, a kettle type reactor, and a devolatilizing device comprising a heat exchanger and a devolatilizing vessel, and further a vented extruder, which is suitable for the cyclocondensation reaction to be carried out using the devolatilizing process together at the same time, can also be used. In these reactors, an autoclave and a kettle type reactor may be particularly preferable. However, even when a reactor such as a vented extruder is used, the

cyclocondensation reaction can be carried out in the reaction state similarly to that in an autoclave and a kettle type reactor by making the venting condition mild, ceasing the venting, or adjusting the temperature condition, the barrel condition, the screw shape, or the operating condition of the screw.

When the cyclocondensation reaction is carried out in advance before the cyclocondensation reaction conducted using the devolatilizing process together at the same time, the methods may include (i) the mixture containing the polymer (a) obtained in the polymerization process and a solvent is reacted by adding a catalyst and heating, (ii) the mixture is reacted by heating without using a catalyst, and (iii) the above (i) or (ii) step is carried out under pressure.

The phrase "the mixture containing the polymer (a) and a solvent" to be introduced into the cyclocondensation reaction in the lactone cyclocondensation process means the polymerization reaction mixture itself obtained in the polymerization process, or the mixture obtained as follows: after the solvent is once removed from the polymerization reaction mixture, a solvent suitable for the cyclocondensation reaction is added again.

A solvent to be added again when the cyclocondensation reaction is carried out in advance before the cyclocondensation reaction using the devolatilizing process together at the same time is not particularly limited, but may include aromatic hydrocarbons such as toluene, xylene, and ethylbenzene; ketones such as methyl ethyl ketone and methyl isobutyl ketone; chloroform, dimethyl sulfoxide, and tetrahydrofuran. These solvents may be used alone, but two or more kinds of them may also be used in combination. It may be preferable to use the same kind of a solvent as that used in the polymerization process.

Although the catalyst to be added in the method (i) may include an esterification catalyst or a transesterification catalyst, such as p-toluenesulfonic acid which is generally used, basic compounds, organocarboxylates, and carbonates, it may be preferable to use the above organophosphorous compounds in the present invention. The addition timing of the catalyst is not particularly limited, but for example, the catalyst may be added at the initial stage of the reaction, may be added while reacting, and may be added by both timing. Although the amount of the catalyst added is not particularly limited, for example, the amount may preferably be 0.001% to 5% by mass, more preferably 0.01% to 2.5% by mass, still more preferably 0.01% to 1% by mass, and particularly preferably 0.05% to 0.5% by mass, relative to the mass of the polymer (a). Although the heating temperature and the heating time in the method (i) are not particularly limited, for example, the heating temperature may preferably be room temperature to 180°C, and more preferably 50°C to 150°C, and the heating time may preferably be 1 to 20 hours, and more preferably 2 to 10 hours. When the heating temperature is lower than room temperature or the heating time is shorter than one hour, the reaction rate in the cyclocondensation reaction may be decreased. In contrast, when the heating temperature is higher than 180°C or the heating time is longer than 20 hours, the resin may be colored or decomposed.

In the method (ii), the polymerization reaction mixture obtained in the polymerization process may be heated as it is using a pressure resistant kettle type reactor, or the like. Although the heating temperature and the heating time in the method (ii) are not particularly limited, for example, the heating temperature may preferably be 100°C to 180°C, and more preferably 100°C to 150°C, and the heating time may preferably be 1 to 20 hours, and more preferably 2 to 10 hours. When the heating temperature is lower than 100°C or the heating time is shorter than one hour, the reaction rate in the cyclocondensation reaction may be decreased. In contrast, when the heating temperature is higher than 180°C or the heating time is longer than 20 hours, the resin may be colored or decomposed.

In either method, there is no problem even if the reaction is carried out under pressure depending on the condition.

When the cyclocondensation reaction is carried out in advance before the cyclocondensation reaction using the devolatilizing process together at the same time, there is no problem even if a part of the solvent volatilizes naturally during the reaction.

The mass decreasing rate in the range of from 150°C to 300°C in the dynamic TG measurement at the completion of the cyclocondensation reaction which is carried out in advance before the cyclocondensation reaction using the devolatilizing process together at the same time, that is, just before the start of the devolatilizing process may preferably be 2% or less, more preferably 1.5% or less, and still more preferably 1% or less. When the mass decreasing rate is greater than 2%, even if the cyclocondensation reaction using the devolatilizing process together at the same time is subsequently carried out, the reaction rate in the cyclocondensation reaction does not rise to a high level enough and the physical properties of the lactone ring-containing polymer obtained may be deteriorated. Further, when the above cyclocondensation reaction is carried out, in addition to the polymer (a), other thermoplastic resin may be allowed to coexist.

In case of the pattern that the reaction rate in the cyclocondensation reaction is raised beforehand to some degree by the cyclocondensation reaction of a hydroxy group and an ester group existing in the molecular chain of the polymer (a) obtained in the polymerization process, and then the cyclocondensation reaction is carried out using the devolatilizing process together at the same time, the polymer (the polymer in which at least parts of the hydroxy group and the ester group existing in the molecular chain have been cyclocondensed) obtained by the cyclocondensation reaction which is carried out in advance and the solvent may directly be introduced into the cyclocondensation reaction using the devolatilizing process together at the same time. Alternatively, if necessary, it is no problem that after the

above polymer (the polymer in which at least parts of the hydroxy group and the ester group existing in the molecular chain have been cyclocondensed) is isolated, the polymer is introduced into the cyclocondensation reaction using the devolatilizing process together at the same time via other treatments such as adding again the solvent.

The devolatilizing process is not limited to be completed at the same time as the cyclocondensation reaction, and it is no problem that the devolatilizing process is completed at some interval after the completion of the cyclocondensation.

<<2-Hydroxymethylacrylic acid ester>>

The 2-hydroxymethylacrylic acid ester used in the present invention is 2-hydroxymethylacrylic acid ester of formula (2):

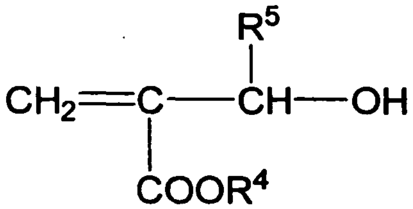

(2)

wherein $R^4$ and $R^5$ each independently means a hydrogen atom or an organic residue having 1 to 20 carbon atoms, in which the organic residue may contain an oxygen atom (s), the turbidity of the ester after heating test at 90°C for two hours being 0.05 or lower. As for the 2-hydroxymethylacrylic acid ester used in the present invention, the turbidity after heating test at 90°C for two hours may preferably be 0.02 or lower, and more preferably 0.01 or lower.

The measurement of turbidity will be carried out as follows: First, 10 g of 2-hydroxymethylacrylic acid ester is put in a 20-mL glass screw tube, to which 500 ppm of hydroquinone monomethyl ether is added as a polymerization inhibitor, and the screw tube is covered with the lid. This screw tube is put into a constant-temperature bath kept at 90°C, and the heating test is carried out for two hours. After two hours pass, the screw tube is taken out and cooled to room temperature, and the absorbance (turbidity) of the objective substance is measured, after the absorbance has been adjusted to be zero with purified water, by setting the wavelength of the spectrophotometer (UV-1650PC, available from Shimadzu Corporation) to 400 nm.

The producing method and the purifying method of 2-hydroxymethylacrylic acid ester of the above formula (2) will hereinafter be described.

The 2-hydroxymethylacrylic acid ester of the above formula (2) can be produced by allowing an acrylic acid ester of formula (4):

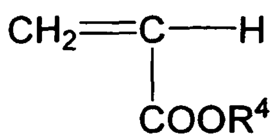

(4)

wherein $R^4$ means a hydrogen atom or an organic residue having 1 to 20 carbon atoms, in which the organic residue may contain an oxygen atom (s), to react with an aldehyde compound in the presence of a tertiary amine compound and water enough to form an aqueous phase at the completion of the reaction.

The acrylic acid ester of the above formula (4) may include: (a) acrylic acid in which the substituent group shown by $R^4$ is a hydrogen atom; (b) acrylic acid alkyl esters in which the substituent group shown by $R^4$ is an alkyl group having 1 to 18 carbon atoms may include methyl acrylate, ethyl acrylate, n-propyl acrylate, isopropyl acrylate, n-butyl acrylate, isobutyl acrylate, t-butyl acrylate, n-octyl acrylate, isooctyl acrylate, 2-ethylhexyl acrylate, lauryl acrylate, and stearyl acrylate; (c) acrylic acid cycloalkyl esters in which the substituent group shown by $R^4$ is a cycloalkyl group having 3 to 10 carbon atoms may include cyclopentyl acrylate and cyclohexyl acrylate; and (d) acrylic acid aryl esters in which the substituent group shown by $R^4$ is an aryl group may include phenyl acrylate, o-methoxyphenyl acrylate, p-methoxyphenyl acrylate, p-nitrophenyl acrylate, o-methylphenyl acrylate, p-methylphenyl acrylate, and p-t-butylphenyl acrylate. In these acrylic acid esters, methyl acrylate, ethyl acrylate, n-butyl acrylate, and 2-ethylhexyl acrylate are particularly suitable.

The aldehyde compound may include aldehyde group-containing compounds; trioxane; paracetaldehyde, and

oxymethylene compounds of formula (5) :

$$HO(CH_2O)_pY \qquad (5)$$

wherein Y means a hydrogen atom, a linear or branched alkyl group having 1 to 8 carbon atoms, or a cycloalkyl group having 3 to 10 carbon atoms, and p means an integer of 1 to 100. Further, when the substituent group shown by Y in the above formula (5) is a cycloalkyl group having 3 to 10 carbon atoms, the cycloalkyl group may contain another different substituent group.

The aldehyde group-containing compounds may include formaldehyde, acetaldehyde, propionaldehyde, butylaldehyde, valeraldehyde, isobutylaldehyde, pivalaldehyde, cyclohexylaldehyde, cyclohexenealdehyde, benzaldehyde, tolualdehyde, anisaldehyde, and furfural.

The oxymethylene compounds of the above formula (5) may include paraformaldehyde which is a polymer (e.g., dimer to hectomer) of formaldehyde, 20% to 50% by mass aqueous solutions of formaldehyde (hydrated formaldehyde), and 20% to 50% by mass aqueous methanol solutions of formaldehyde.

In these aldehyde compounds, acetaldehyde, paraformaldehyde, 20% to 50% by mass aqueous solutions of formaldehyde, and 20% to 50% by mass aqueous methanol solutions of formaldehyde are particularly suitable. The aldehyde compounds may be used alone, or two or more kinds of them may also be used in combination.

The 2-hydroxymethylacrylic acid ester of the above formula (2) can be obtained by allowing an acrylic acid ester of the above formula (4) to react with an aldehyde compound. In the above formula (2), the substituent group shown by $R^5$ is a substituent group derived from the aldehyde compound.

The tertiary amine compound used as a catalyst in the above reaction may include trialkylamines such as trimethylamine, triethylamine, tri-n-propylamine, and triisopropylamine; N,N-dimethylalkylamine such as N,N-dimethylethylamine, N,N-dimethylpropylamine, N,N-dimethylisopropylamine, N,N-dimethylbutylamine, N,N-dimethylisobutylamine, and N,N-dimethy-t-butylamine, N,N-dimethyl(trimethysilyl)amine; and N,N-diethylalkylamine such as N, N-diethylmethylamine, N,N-diethylpropylamine, and N,N-diethylisopropylamine. These tertiary amine compounds may be used alone, or two or more kinds of them may also be used in combination. In these tertiary amine compounds, compounds having relatively high solubility in water may be preferable, N-methylalkylamines (N-methyl compounds) having boiling points of 100°C or lower at normal pressure and having at least one N-methyl group may be more preferable, N,N-dimethylalkylamines having boiling points of 100°C or lower at normal pressure and having two N-methyl groups may be still more preferable, and trimethylamine may be particularly preferable.

Although tertiary amine compounds can be used in various states such as liquid and gassy states, it is preferable to use as a 5% to 80% by mass aqueous solution, and more preferable to use as a 20% to 60% by mass aqueous solution. By using the tertiary amine compound in the state of an.aqueous solution, it becomes easy to handle the tertiary amine compound at the time of starting the reaction and during the reaction, and it is also easy to handle the tertiary amine compound for recovering the tertiary amine compound after the completion of the reaction and for reusing the tertiary amine compound.

Although the molar ratio of the acrylic acid ester of the above formula (4) and the aldehyde compound (i.e., the acrylic acid ester of the above formula (4) / the aldehyde compound) is not particularly limited, the ratio may preferably be in the range of at least 2 or higher, more preferably from 2.5 to 15, and still more preferably from 2.5 to 8. When the molar ratio is smaller than 2, the generation of impurities derived from the aldehyde compound may increase, the selectivity to the 2-hydroxymethylacrylic acid ester of the above formula (2) may be decreased, and a lot of labors may be required for its purification.

Although the molar ratio of the tertiary amine compound and the aldehyde compound (i.e., the tertiary amine compound / the aldehyde compound) is not particularly limited, the ratio may preferably be in the range of from 0.01 to 2, more preferably from 0.02 to 1, and still more preferably from 0.05 to 0.8. When the molar ratio is smaller than 0.01, the improvement of the reaction speed may be unable to be observed, the generation of by-products may increase, and the selectivity to 2-hydroxymethylacrylic acid ester of the above-mentioned formula (2) may be decreased. In contrast, when the molar ratio is greater than 2, the hydrolysis of the acrylic acid ester of the above formula (4), which is a starting material, or the hydrolysis of the 2-hydroxymethylacrylic acid ester of the above formula (2), which is the product, may occur, although it depends on the reaction conditions.

The acrylic acid ester of the above formula (4) and the aldehyde compound are allowed to react in the presence of water enough to form an aqueous phase at the completion of the reaction. Preferably, they may be allowed to react in the presence of such an amount of water that through the beginning of the reaction, during the reaction, and the completion of the reaction, the reaction can progress while the reaction solution (the reaction system) forms two phases system of an organic phase and an aqueous phase. Further, the organic phase means a mixture containing compounds substantially insoluble in water, that is, the acrylic acid ester of the above formula (4), which is a starting material, the 2-hydroxymethylacrylic acid ester of the above formula (2), which is the product, and a solvent described later.

The amount of water to be used is not particularly limited, but has only to be properly set so as to give the

optimum amount, taking the following matters into consideration: the kinds (properties), combination, and amounts of the materials to be used such as an acrylic acid ester of the above formula (4), an aldehyde compound, a tertiary amine compound, and a solvent; the properties of a 2-hydroxymethylacrylic acid ester of the above formula (2), which is to be obtained; the reaction conditions such as reaction temperature; and the like. Specifically, for example, when methyl acrylate is used as an acrylic acid ester of the above formula (4), paraformaldehyde is used as an aldehyde compound, and trimethylamine is used as a tertiary amine compound, water has to be added, although it also depends on the reaction condition, so that the amount of water may become about 2% to 40% by mass, relative to the total amount of the methyl acrylate and the paraformaldehyde. By adding such an amount of water, through the beginning of the reaction, during the reaction, and the completion of the reaction, the reaction solution forms two phases system of an organic phase and an aqueous phase, and the reaction progresses effectively.

At the time of the reaction, a solvent insoluble in water can be used, if necessary, to form an organic phase. The kind of solvent is not particularly limited, so long as the solvent dissolves the acrylic acid ester of the above formula (4), the aldehyde compound, and the 2-hydroxymethylacrylic acid ester of the above formula (2), and is an inert compound to the reaction. The amount of solvent to be used is not particularly limited, but has only to be properly set so as to give the optimum amount, taking the following matters into consideration: the kinds (properties), combination, and amounts of the materials to be used such as an acrylic acid of the above formula (4), an aldehyde compound, a tertiary amine compound, and a solvent; the properties of a 2-hydroxymethylacrylic acid ester of the above formula (2), which is to be obtained; the reaction conditions such as reaction temperature; and the like. The solvent may be used alone, or two or more kinds of solvents may also be used in combination. Further, the acrylic acid ester of the above formula (4) can also be utilized as a solvent by using an excessively large amount.

Although the reaction conditions and the like when the above reaction is carried out are not particularly limited, the acrylic acid ester of the above formula (4), which is a starting material, and the 2-hydroxymethylacrylic acid ester of the above formula (2), which is the product, each have a polymerizable double bond in the molecule, so that they have the property of being polymerized easily. Accordingly, when the acrylic acid ester of the above formula (4) is allowed to react with the aldehyde compound, in order to suppress the polymerization of these compounds, it may be preferable to add a polymerization inhibitor or molecular oxygen in the reaction system.

The polymerization inhibitor is not particularly limited, but may include quinones such as hydroquinone, methylhydroquinone, t-butylhydroquinone, 2,4-di-t-butylhydroquinone, and 2,4-dimethylhydroquinone; amine compounds such as phenothiazine; and phenols such as 2,4-dimethyl-6-t-butylphenol, 2,4-di-t-butylphenol, and p-methoxyphenol. These polymerization inhibitors may be used alone, or two or more kinds of them may also be used in combination. Although the amount of polymerization inhibitor to be added is not particularly limited, for example, the ratio of the polymerization inhibitor to the acrylic acid ester of the above formula (4) has only to be set so as to be in the range of from 0.001% to 1% by mass.

As the molecular oxygen, for example, air, or a mixed gas of molecular oxygen, nitrogen, and the like can be used. In this case, in order to allow molecular oxygen to be contained in the reaction solution, that is, the organic phase or the aqueous phase, the solution has only to be stirred or the gas containing molecular oxygen has only to be blown into the solution (i.e., "bubbling"). It may be preferable to use a polymerization inhibitor and molecular oxygen together to suppress polymerization enough.

Although the reaction temperature is not particularly limited, the reaction temperature may preferably be in the range of from 10°C to 150°C, more preferably from 40°C to 100°C, and still preferably from 40°C to 80°C, in order to suppress polymerization. When the reaction temperature is lower than 10°C, the reaction speed may become slow, so that the reaction time may become too long and the 2-hydroxymethylacrylic acid ester of the above formula (2) may be unable to be produced effectively. In contrast, when the reaction temperature is higher than 150°C, it may become impossible to suppress polymerization, and the hydrolysis of the acrylic acid ester of the above formula (4) may occur.

The reaction time has only to be properly set depending on the reaction temperature, the kinds (properties), combination, and amounts of the materials to be used such as an acrylic acid ester of the above formula (4), an aldehyde compound, a tertiary amine compound, and a solvent, so that the above reaction is completed. Therefore, although the reaction time is not particularly limited, it is sufficient to be about 0.5 to 10 hours. Moreover, the reaction pressure is not particularly limited, but any of normal pressure (e.g., atmospheric pressure), reduced pressure, and increased pressure may be used.

After the completion of the reaction, the reaction solution is separated into the organic phase and the aqueous phase by the prescribed operation such as liquid separation. After sufficiently washing the organic phase with water, if necessary, the unreacted acrylic acid ester of the above formula (4) and the solvent are separated and recovered by the fractional distillation. After heat treating at a prescribed temperature while refluxing, the high purity 2-hydroxymethyl acrylate of the above formula (2) can simply be obtained by rectification. In general, the crude 2-hydroxymethylacrylic acid ester of the above formula (2), which was obtained by allowing an acrylic acid ester of the above formula (4) to react with an aldehyde compound, contains more than one by-product, although the amounts of them are small. Some of the by-products are such impurities that differences in boiling point between them and the objective 2-hydroxymethylacrylic acid ester of the above formula (2) are small.

The impurities to be heat treated, that is, such impurities that differences in boiling point between them and the objective 2-hydroxymethylacrylic acid ester of the above formula (2) are small, are acetal compounds contained in the crude 2-hydroxymethylacrylic acid ester of the above formula (2). These acetal compounds may include ethyl 2-ethoxymethoxymethylacrylate when the objective substance is ethyl 2-hydroxymethylacrylate.

The above acetal compounds have small differences in boiling point between them and the objective 2-hydroxymethylacrylic acid ester of the above formula (2), so that they are difficult to be separated by distillation as they are. However, the present inventors have extensively studied and as a result have found that the above acetal compounds can easily be changed, by heat treatment, into the compounds having boiling points higher than that of the 2-hydroxymethylacrylic acid ester of the above formula (2).

The temperature at which the crude 2-hydroxymethylacrylic acid ester of the above formula (2) is heat treated is the temperature at which acetal compounds contained in it can be changed into the compounds having higher boiling points, and if the temperature is in the range of from -10°C to 30°C, relative to the bottom temperature at the time of the rectification (i.e., in the range of from a temperature 10°C lower than, to a temperature 30°C higher than, the bottom temperature at the time of the rectification; the phrase "relative to the bottom temperature at the time of the rectification" will hereinafter be used in such a meaning), no white foreign matters will be generated in the obtained 2-hydroxymethylacrylic acid ester of the above formula (2) during the storage. Further, the heat treating temperature may preferably be in the range of from 0°C to 25°C, relative to the bottom temperature at the time of the rectification, and more preferably in the range of from 10°C to 20°C, relative to the bottom temperature at the time of the rectification, for effective treatment. When the heat treating temperature is lower than -10°C, relative to the bottom temperature at the time of the rectification, white foreign matters may easily be generated in the obtained 2-hydroxymethylacrylic acid ester of the above formula (2) during the storage. Moreover, the 2-hydroxymethylacrylic acid ester of the above formula (2) has a polymerizable double bond in the molecule, so that it has the property of being polymerized easily. For this reason, when the heat treating temperature is higher than 30°C, relative to the bottom temperature at the time of the rectification, it may become impossible to suppress polymerization.

Further, the heat treatment may preferably be carried out while boiling under vacuum. The 2-hydroxymethylacrylic acid ester of the above formula (2) is boiled and distilled out, but is cooled in a condenser to be returned in a distillation apparatus. The degree of vacuum at this time has only to be pressure under which it is boiled at a temperature in the range of from -10°C to 30°C, relative to the bottom temperature at the time of the rectification.

The heat treating time has only to be set properly depending on the heat treating temperature and is not particularly limited, but the heat treating time is usually in the range of from 0.5 to 5 hours.

Moreover, the 2-hydroxymethylacrylic acid ester of the above formula (2) has the property of being polymerized easily, so that when the heat treatment is carried out, it may be preferable to add a polymerization inhibitor or molecular oxygen in order to suppress the polymerization of this compound.

The polymerization inhibitor is not particularly limited, but may include polymerization inhibitors such as described above as those which can be used in case of allowing an acrylic acid ester of the above formula (4) to react with an aldehyde compound. The polymerization inhibitors may be used alone, or two or more kinds of them may also be used in combination. Although the amount of polymerization inhibitor to be added is not particularly limited, for example, the ratio of the polymerization inhibitor to the crude 2-hydroxymethylacrylic acid ester of the above formula (2) has only to be set so as to be in the range of from 0.01% to 1% by mass.

As molecular oxygen, for example, air, or a mixed gas of molecular oxygen, nitrogen, and the like can be used. In this case, the gas containing molecular oxygen has only to be blown into the reaction solution, that is, the organic phase or the aqueous phase (i.e., "bubbling"). It may be preferable to use a polymerization inhibitor and molecular oxygen together to suppress polymerization enough.

In this manner, when the crude 2-hydroxymethylacrylic acid ester of the above formula (2), which is obtained by allowing an acrylic acid ester of the above formula (4) to react with an aldehyde compound, is purified, the high purity 2-hydroxymethylacrylic acid ester of the above formula (2) can be obtained by rectification after heat treatment at a temperature in the range of from -10°C to 30°C, relative to the bottom temperature at the time of the rectification. By the above heat treatment, acetal compounds which are impurities having small differences in boiling point between them and the objective 2-hydroxymethylacrylic acid ester of the above formula (2) are changed into the compounds having higher boiling points than that of the 2-hydroxymethylacrylic acid ester of the above formula (2). At this time, because low boiling point components are also generated, the components, which become the factor of white turbidity when rectifying the 2-hydroxymethylacrylic acid ester of the above formula (2), can be removed by heat treatment at a high temperature before the rectification. For this reason, the high quality 2-hydroxymethylacrylic acid ester of the above formula (2) can be obtained in high yield.

As described above, when the lactone ring-containing polymer is produced, the use of such a high quality 2-hydroxymethylacrylic acid ester of the above formula (2) as a monomer and the use of a t-amyl type peroxide as a polymerization initiator make it possible to very effectively suppress the generation of polymer gels in the lactone ring-containing polymer.

<<Applications and forming of lactone ring-containing polymer>>

The lactone ring-containing polymer of the present invention is suitable particularly for use in optical applications because the polymer is such a forming material that foreign matters are very few and gelation does not easily occur, in addition to being excellent in transparency and heat resistance as well as having the desired properties such as low coloring, mechanical strength, and forming and processing properties. The lactone ring-containing polymer of the present invention is useful for optical components including, for example, a light guide material, an optical lens, an optical film, an optical prism, and an optical disk. In these optical components, a light guide material, an optical lens, an optical film, and the like may be particularly preferable.

The lactone ring-containing polymer of the present invention can be formed into various shapes depending on the applications. Usually, the lactone ring-containing polymer of the present invention is first heated and granulated in, for example, forming materials such as pellets, and then are secondary formed into various shapes. Formable shapes may include films, sheets, plates, disks, blocks, balls, lenses, rods, strands, and codes. The forming method has only to be properly selected, depending on the shapes, from various forming methods known so far, and is not particularly limited.

Using an optical film, which is a particularly preferable application, as one example, the method of producing an optical film from the lactone ring-containing polymer of the present invention will hereinafter be described in detail.

<Production of optical film>

To produce an optical film from the lactone ring-containing polymer of the present invention, after the starting materials of a film are pre-blended in a known mixer such as an omni mixer, the obtained mixture is extruded and kneaded. In this case, the mixer used for extruding and kneading is not particularly limited, and known mixers including, for example, extruders such as single screw extruders and twin screw extruders, and pressure kneaders can be used.

As the method for forming a film, there can be mentioned known film forming methods such as solution casting method, melt extrusion method, calender method, and compression forming method. In these film forming methods, the solution casting method and the melt extrusion method may be particularly preferable.

The solvent to be used in the solution casting method may include aromatic hydrocarbons such as benzene, toluene, and xylene; aliphatic hydrocarbons such as cyclohexane and decalin; esters such as ethyl acetate and butyl acetate; ketones such as acetone, methyl ethyl ketone, and methyl isobutyl ketone; alcohols such as methanol, ethanol, isopropanol, butanol, isobutanol, methyl cellosolve, ethyl cellosolve, and butyl cellosolve; ethers such as tetrahydrofuran and dioxane; halogenated hydrocarbons such as dichloromethane, chloroform, and carbon tetrachloride; dimethylformamide; dimethyl sulfoxide. These solvents may be used alone, or two or more kinds of them may also be used in combination.

The apparatus for carrying out the solution casting method may include drum type casting machines, band type casting machines, and spin coaters.

The melt extrusion methods may include T die method and inflation method. The forming temperature in that case has only to be adjusted properly depending on the glass transition temperature of the starting material of a film and is not particularly limited. However, the temperature may preferably be 150°C to 350°C, and more preferably 200°C to 300°C.

When a film is formed by the T die method, a T die is installed on the apical end of the known single screw extruder or twin screw extruder, and a roll-shaped film can be obtained by winding up the film extruded in film shape. At this time, the uniaxial drawing of the film can be carried out by properly adjusting the winding up temperature and drawing the film in the extrusion direction. Further, by drawing the film in the direction perpendicular to the extrusion direction, the simultaneous biaxial drawing, the sequential biaxial drawing, and the like can also be carried out.

The film made of the lactone ring-containing polymer of the present invention may be either an undrawn film or a drawn film. In case of a drawn film, the film may be either a uniaxially drawn film or a biaxially drawn film. In case of a biaxially drawn film, the film may be either a simultaneously biaxially drawn film or a sequentially biaxially drawn film. When a film is biaxially drawn, the mechanical strength of the film is improved, and the film performance is improved. As for the lactone ring-containing polymer of the present invention, even if a film is drawn, an increase in retardation can be suppressed by mixing any of other thermoplastic resins, and a film maintaining the optical isotropy can be obtained.

The drawing temperature may be preferable to be near the glass transition temperature of the lactone ring-containing polymer, which is the starting material of a film. Specifically, the drawing temperature may preferably be in the range of from (the glass transition temperature - 30°C) to (the glass transition temperature + 100°C), and more preferably in the range of from (the glass transition temperature - 20°C) to (the glass transition temperature + 80°C). When the drawing temperature is lower than (the glass transition temperature - 30°C), a sufficient draw ratio may be

unable to be obtained. In contrast, when the drawing temperature is higher than (the glass transition temperature + 100°C), the flow of the polymer may occur, so that it may become impossible to carry out stable drawing.

The draw ratio defined by the area ratio may preferably be in the range of from 1.1 to 25 times, and more preferably in the range of from 1.3 to 10 times. When the draw ratio is smaller than 1.1 times, an improvement in toughness resulting from drawing may be unable to be attained. In contrast, when the draw ratio is greater than 25 times, the effect of raising the draw ratio may be unable to be observed.

The drawing speed may preferably be in the range of from 10%/min. to 20,000%/min. in one direction, and more preferably in the range of from 100%/min. to 10,000%/min. When the drawing speed is lower than 10%/min., it may take time to obtain an enough draw ratio, and the producing cost may become high. In contrast, when the drawing speed is higher than 20,000%/min., the breaking of a drawn film and the like may occur.

The film made of the lactone ring-containing polymer of the present invention can be subjected to heat treatment (annealing) and the like after drawing treatment to stabilize the optical isotropy and the mechanical properties. The heat treatment conditions have only to be properly selected similarly to those of the heat treatment for known drawn films, and are not particularly limited.

The thickness of the film made of the lactone ring-containing polymer of the present invention may preferably be 5 to 200 μm, and more preferably 10 to 100 μm. When the thickness is smaller than 5 μm, the strength of the film decreases, and when the durability test is carried out in the state of being stuck to other parts, crimp may be increased. In contrast, when the thickness is greater than 200 μm, the transparency of the film is decreased, and the moisture permeability of the film becomes small, and when a water type adhesive is used for being stuck to other parts, the drying speed of water as the solvent therefor may be decreased.

The surface wet tensile force of the film made of the lactone ring-containing polymer of the present invention may preferably be 40 mN/m or higher, more preferably 50 mN/m or higher, and still more preferably 55 mN/m or higher. When the surface wet tensile force is at least 40 mN/m or higher, the adhesive strength of the film made of the lactone ring-containing polymer of the present invention with other parts is further improved. In order to adjust the surface wet tensile force, for example, the corona discharge treatment, the ozone spraying, the ultraviolet irradiation, the flame treatment, the chemical treatment, and other surface treatments known so far can be carried out.

The film made of the lactone ring-containing polymer of the present invention may contain various additives. The additives may include antioxidants such as hindered phenol antioxidants, phosphorous antioxidants, and sulfuric antioxidants; stabilizers such as light resistance stabilizers, weather resistance stabilizers, and thermostabilizers; reinforcing materials such as glass fiber and carbon fiber; ultraviolet absorbers such as phenyl salicylate, (2,2'-hydroxy-5-methylphenyl)benzotriazole, and 2-hydroxybenzophenone; near-infrared absorbers; flame retardants such as tris(dibromopropyl) phosphate, triallyl phosphate, and antimony oxide; antistatic agents such as anionic surfactants, cationic surfactants, and nonionic surfactants; colorants such as inorganic pigments, organic pigments, and dyes; organic fillers and inorganic fillers; resin modifiers; plasticizers; lubricants; and antistatic agents.

The contents of additives in a thermoplastic resin film composed mainly of the lactone ring-containing polymer may preferably be 0% to 5% by mass, more preferably 0% to 2% by mass, and still more preferably 0% to 0.5% by mass.

Examples

The present invention will be described below in detail by reference to Examples and Comparative Examples, but the present invention is not limited to these Examples. The present invention can be put into practice after appropriate modifications or variations within a range meeting the gists described above and later, all of which are included in the technical scope of the present invention.

First, the evaluating method of the lactone ring-containing polymer will be described.

<Polymerization reaction rate and polymer composition analysis>

The reaction rate in the polymerization reaction and the content of a specific monomer unit in the polymer were determined by measuring the amount of unreacted monomer in the obtained polymerization reaction mixture with the use of a gas chromatograph (GC 17A, available from Shimadzu Corporation).

<Dynamic TG>

After the polymer (or the polymer solution or the pellet) was dissolved in or diluted with tetrahydrofuran, the solution was put into excessive hexane or methanol to cause the reprecipitation of the polymer. The precipitate was taken out and dried under vacuum (under a pressure of 1.33 hPa (1 mmHg) and at 80°C for 3 hours or longer) to remove the volatile components and the like. The obtained white solid resin was analyzed by the following method (the dynamic TG method).

Measuring apparatus: differential thermal balance (Thermo Plus 2 TG-8120 Dynamic TG, available from Rigaku Co., Ltd.);
Measuring condition: the amount of sample was 5 to 10 mg;
Temperature rising speed: 10°C/min.;
Ambient atmosphere: nitrogen flow of 200 mL/min.;
Method: step-wise isothermal control method (controlling the value of the mass decreasing speed to be 0.005%/s or lower in the range of from 60°C to 500°C)

<Lactone cyclizing rate>

As an example, in case of the polymer composition obtained from methyl methacrylate and 2-hycroxymethylmethyl acrylate, the reaction rate in the dealcoholization can be determined from the mass decrease by the dealcoholization reaction from 150°C before the mass decrease begins to 300°C before the polymer begins to decompose, in the dynamic TG measurement on the basis of the mass decreasing amount caused when all hydroxy groups are dealcoholized as methanol.

That is, in the dynamic TG measurement of the polymer having the lactone ring structure, the mass decreasing rate between 150°C and 300°C is measured, and the obtained actual measurement value is assumed to be the measured mass decreasing rate (X). On the other hand, the mass decreasing rate from the above polymer composition when assuming that all hydroxy groups contained in the polymer composition will be changed into an alcohol and dealcoholized to take part in the formation of the lactone ring (that is, the mass decreasing rate calculated assuming that 100% of dealcoholization reaction occurred on the composition) is assumed to be the theoretical mass decreasing rate (Y). Further, more specifically, the theoretical mass decreasing rate (Y) can be calculated from the molar ratio of the starting material monomer having the structure (the hydroxy group) taking part in the dealcoholization reaction in the polymer, that is, the rate of content of the starting material monomer in the above polymer composition. These values are substituted for the dealcoholization calculating formula:

$$1 - (\text{the measured mass decreasing rate (X)} / \text{the theoretical mass decreasing rate (Y)})$$

and the calculated value is written by the percentage (%), and thus the reaction rate in the dealcoholization reaction (the lactone cyclizing rate) can be obtained.

As an example, the rate of content of the lactone ring structure in the pellets obtained in Example 1 described later is calculated. When the theoretical mass decreasing rate (Y) in this case is calculated, because the molecular weight of methanol is 32, the molecular weight of methyl 2-hydroxymethylacrylate is 116, and the rate of content (mass ratio) of methyl 2-hydroxymethylacrylate in the polymer is 20.0% by mass from the composition, the value becomes (32/116) × 20.0% by mass ≅ 5. 52% by mass. On the other hand, the measured mass decreasing rate (X) from the dynamic TG measurement was 0.17% by mass. These values are substituted for the above dealcoholization calculating formula, the value becomes 1 - (0.17/5.52) ≅ 0.969, the reaction rate in the dealcoholization (the lactone cycling rate) is 96.9%.

<Weight average molecular weight>

The weight average molecular weight of a polymer was determined by polystyrene calibration using a gel permeation chromatograph (GPC system, available from Tosoh Corporation). As the developing solution, chloroform was used.

<Thermal analysis of polymer>

The thermal analysis of a polymer was carried out using a differential scanning calorimeter (DSC-8230, available from Rigaku Co., Ltd.) on the conditions that the amount of sample was about 10 mg, the temperature rising speed was 10°C/min., and the nitrogen flow was 50 mL/min. Further, the glass transition temperature (Tg) was determined by the midpoint method in accordance with ASTM-D-3418.

<Melt flow rate>

The melt flow rate was measured at the examination temperature of 240°C and under the load of 10 kg in

accordance with JIS-K6874.

<Measurement of melt viscosity>

The melt viscosity of a polymer was measured using a flow tester (CFT-500C, available Shimadzu Corporation) on the conditions that the temperature was 250°C, the load was 10 $kgf/cm^2$, and the die shape was 0.5 mmϕ × 1 mm.

<Content of polymer gels>

First, 100 g of a polymer was dissolved in 500 mL of methyl ethyl ketone purified by microfiltration. The obtained polymer solution was then passed through a membrane filter made of polytetrafluoroethylene and of 1.0 μm in average pore size, and polymer gels were obtained on the filter. In the obtained polymer gels, those of 50 μm or greater in average particle diameter were counted by the eyes under a microscope and shown as the number of pieces per 100 g of the polymer.

Next, the synthetic example of methyl 2-hydroxymethylacrylate will be described. Further, in Synthetic Example 1, heat treatment was carried out at a temperature higher than that within a distillation still at the time of fractional distillation by 0°C to 26.5°C, and high purity methyl 2-hydroxymethylacrylate was obtained. In Synthetic Example 2, heat treatment was carried out at a temperature lower than that within a distillation still at the time of fractional distillation by 2°C to 30°C, and low purity methyl 2-hydroxymethylacrylate was obtained.

<Synthetic Example 1>

A 3-L four-neck flask equipped with a thermometer, a gas blowing tube, a condenser, a stirring device, and a water bath, was charged with 2,066 g (24 moles) of methyl acrylate as an acrylic acid ester of the above formula (4), 195.8 g (6 moles) of 92% by mass paraformaldehyde as an aldehyde compound, 237.8 g (1.2 moles) of 30% by mass trimethyl amine aqueous solution as a tertiary amine compound, and 2.1 g of p-methoxyphenol as a polymerization inhibitor. The proportion of p-methoxyphenol to methyl acrylate was 1,000 ppm. Then, the reaction solution was stirred at 70°C for 8 hours while blowing air into the solution, thereby causing reaction.

After the completion of the reaction, the reaction solution was transferred to a separating funnel and separated into an organic phase and an aqueous phase. Then, the organic phase was washed with 100 g of water added thereto. After the solution was separated into an organic phase and an aqueous phase, the organic phase was further washed with the equal amount of water and separated into an organic phase and an aqueous phase.

The obtained organic phase was transferred in a 2-L four-neck flask equipped with a thermometer, a gas blowing tube, an empty tower distillation tube, a stirring device, and an oil bath, to which 5 g of phenothiazine was added as a stabilizer, and methyl acrylate was recovered under a pressure of 400 to 133 hPa (300 to 100 mmHg) while blowing air and adjusting the temperature within the flask not to exceed 100°C.

Then, the pressure was set to be 40 hPa (30 mmHg), and heat treatment was carried out while boiling the organic phase at an inner temperature of 110°C to 120°C. The boiled liquid was cooled with the condenser and returned in the flask.

After the heat treatment, the fractional distillation of the organic phase was carried out, and 418 g of methyl 2-hydroxymethylacrylate was obtained as the fraction of distillate at a tower top temperature of 86°C to 87°C/13.3 hPa (10 mmHg). The bottom temperature at the time of the rectification was 93.5°C to 110°C when this objective substance was obtained.

Then, 10 g of methyl 2-hydroxymethylacrylate thus obtained was put in a 20-mL glass screw tube, to which 500 ppm of hydroquinone monomethyl ether was added as a polymerization inhibitor, and the screw tube was covered with the lid. This screw tube was put into a constant-temperature bath kept at 90°C, and the heating test was carried out for two hours. After two hours passed, the screw tube was taken out and cooled, and the absorbance (turbidity) of the objective substance was measured, after the absorbance has been adjusted to be zero with purified water, by setting the wavelength of the spectrophotometer (UV-1650PC, available from Shimadzu Corporation) as 400 nm. The turbidity of this objective substance was 0.001.

This objective substance was stored at 40°C for three months. However, the objective substance was transparent and colorless, and no white foreign matters were generated.

<Synthetic Example 2>

To the step of recovering methyl acrylate, the same operation as that in Synthetic Example 1 was carried out. Then, the pressure was set to be 26.7 hPa (20 mmHg), and heat treatment was carried out while boiling the organic phase at an inner temperature of 100°C to 110°C. The boiled liquid was cooled with the condenser and returned in the flask.

After the heat treatment, the fractional distillation of the organic phase was carried out, and 407 g of methyl 2-hydroxymethylacrylate was obtained as the fraction of distillate at a tower top temperature of 105°C to 106°C/40 hPa (30 mmHg). The bottom temperature at the time of the rectification was 112°C to 130°C when this objective substance was obtained.

Then, 10 g of methyl 2-hydroxymethylacrylate thus obtained was put in a 20-mL glass screw tube, to which 500 ppm of hydroquinone monomethyl ether was added as a polymerization inhibitor, and the screw tube was covered with the lid. This screw tube was put into a constant-temperature bath kept at 90°C, and the heating test was carried out for two hours. After two hours passed, the screw tube was taken out and cooled, and the absorbance (turbidity) of the objective substance was measured, after the absorbance has been adjusted to be zero with the purified water, by setting the wavelength of the spectrophotometer (UV-1650PC, available from Shimadzu Corporation) to 400 nm. The turbidity of this objective substance was 0.072.

This objective substance was stored at 40°C for three months. However, the objective substance became clouded on 2nd month, and white deposits were generated three months later.

<Example 1>

A 30-L kettle type reactor equipped with a stirring device, a temperature sensor, a condenser, and a nitrogen introducing tube was charged with 8,000 g of methyl methacrylate (MMA), 2,000 g of methyl 2-hydroxymethylacrylate (MHMA; prepared in Synthetic Example 1), and 10,000 g of toluene. The solution was heated to 105°C while passing nitrogen through the solution, and 10.0 g of t-amylperoxyisononanoate (Rupasol 570, available from Atofina Yoshitomi, Ltd.) as a polymerization initiator was added when the reflux began, at which same time while dropping a solution containing 20.0 g of t-amylperoxyisononanoate and 100 g of toluene for two hours, the solution polymerization was carried out at about 105°C to 110°C under reflux, and further matured for four hours.

To the obtained polymer solution, 10 g of a mixture of stearyl phosphate/distearyl phosphate (Phoslex A-18, available from Sakai Chemical Industry Co., Ltd.) was added, and cyclocondensation reaction was carried out at about 90°C to 110°C for five hours under reflux. Then, the obtained polymer solution was introduced into a vented twin screw extruder ($\phi$ = 29.75 mm, L/D = 42), in which the barrel temperature was 260°C, the number of revolutions was 100 rpm, the degree of reduced pressure was 13.3 to 400 hPa (10 to 300 mmHg), the number of rear vent was one, and the number of fore vent was four, at the throughput speed of 2.0 kg/h in terms of the amount of resin, and cyclocondensation reaction and devolatilizing were carried out in this extruder, and transparent pellets of a lactone ring-containing polymer were obtained by extrusion.

As for the obtained lactone ring-containing polymer, the dynamic TG measurement was carried out, and 0.17% by mass in mass decrease was detected. Moreover, the characteristics of this lactone ring-containing polymer were as follows: the weight average molecular weight was 147,700, the glass transition temperature was 130°C, the melt flow rate was 11.0 g/10 min., the lactone cyclizing rate was 97%, and the content of polymer gels was 2 pieces/100 g. The results are shown in Table 1.

Moreover, as for the obtained pellets of the lactone ring-containing polymer, when the kneading test was carried out with a twin screw extruder ($\phi$= 29.75 mm, L/D = 30), in which the barrel temperature was 270°C, the number of rotations was 100 rpm, the content of polymer gels remained 2 pieces/100 g. The result is shown in Table 1.

The melt viscosity at 250°C of the obtained pellets of the lactone ring-containing polymer was measured to be 2, 100 poise. Then, after the pellets of the lactone ring-containing polymer were left for 30 minutes in an oven at 280°C, the melt viscosity at 250°C of the pellets of the lactone ring-containing polymer was measured again to be 3,700 poise. From the melt viscosity measurements before and after heating, the increasing rate in viscosity (after heating/before heating) was 1.76. The results are shown in Table 1.

<Example 2>

A 30-L kettle type reactor equipped with a stirring device, a temperature sensor, a condenser, and a nitrogen introduction tube was charged with 8,000 g of methyl methacrylate (MMA), 2,000 g of methyl 2-hydroxymethylacrylate (MHMA; prepared in Synthetic Example 2), 10,000 g of toluene, and 5.0 g of n-dodecyl mercaptan. The solution was heated to 100°C while passing nitrogen through the solution, and 10.0 g of t-amylperoxyisononanoate (Rupasol 570, available from Atofina Yoshitomi Co., Ltd.) as a polymerization initiator was added when the reflux began, at which same time while dropping a solution containing 20.0 g of t-amylperoxyisononanoate and 100 g of toluene for two hours, the solution polymerization was carried out at about 95°C to 110°C under reflux, and further matured for four hours.

To the obtained polymer solution, 10 g of a mixture of stearyl phosphate/distearyl phosphate (Phoslex A-18, available from Sakai Chemical Industry Co., Ltd.) was added, and cyclocondensation reaction was carried out at about 90°C to 110°C for five hours under reflux. Then, in the same manner as described in Example 1, the obtained polymer solution was subjected to cyclocondensation reaction and devolatilizing in an extruder, and transparent pellets of a

lactone ring-containing polymer were obtained by extrusion.

As for the obtained lactone ring-containing polymer, the dynamic TG measurement was carried out, and 0.18% by mass in mass decrease was detected. Moreover, the characteristics of this lactone ring-containing polymer were as follows: the weight average molecular weight was 141,000, the glass transition temperature was 130°C, the melt flow rate was 13.0 g/10 min, the lactone cyclizing rate was 97%, and the content of polymer gels was 58 pieces/100 g. The results are shown in Table 1.

Moreover, as for the obtained pellets of the lactone ring-containing polymer, when the kneading test was carried out on the same conditions as those used in Example 1, the content of polymer gels increased to 60 pieces/100 g. The results are shown in Table 1.

The melt viscosity at 250°C of the obtained pellets of the lactone ring-containing polymer was measured to be 2, 050 poise. Then, after the pellets of the lactone ring-containing polymer were left for 30 minutes in an oven at 280°C, the melt viscosity at 250°C of the pellets of the lactone ring-containing polymer was measured again to be 3,800 poise. From the melt viscosity measurements before and after heating, the increasing rate in viscosity (after heating/before heating) was 1.85. The results are shown in Table 1.

<Example 3>

A 30-L kettle type reactor equipped with a stirring device, a temperature sensor, a condenser, and a nitrogen introduction tube was charged with 8,000 g of methyl methacrylate (MMA), 2,000 g of methyl 2-hydroxymethylacrylate (MHMA; prepared in Synthetic Example 1), and 10,000 g of toluene. The solution was heated to 105°C while passing nitrogen through the solution, and 5.0 g of 1,1'-azobis(cyclohexane-1-carbonitrile) (V-40, available from Wako Pure Chemical Industries, Ltd.) as a polymerization initiator was added when the reflux began, at which same time while dropping a solution containing 10.0 g of 1,1'-azobis(cyclohexane-1-carbonitrile) and 100 g of toluene for two hours, the solution polymerization was carried out at about 105°C to 110°C under reflux, and further matured for four hours.

To the obtained polymer solution, 10 g of a mixture of stearyl phosphate/distearyl phosphate (Phoslex A-18, available from Sakai Chemical Industry Co., Ltd.) was added, and cyclocondensation reaction was carried out at about 90°C to 110°C for five hours under reflux. Then, in the same manner as described in Example 1, the obtained polymer solution was subjected to cyclocondensation reaction and devolatilizing in an extruder, and transparent pellets of a lactone ring-containing polymer were obtained by extrusion.

As for the obtained lactone ring-containing polymer, the dynamic TG measurement was carried out, and 0.31% by mass in mass decrease was detected. Moreover, the characteristics of this lactone ring-containing polymer were as follows: the weight average molecular weight was 168,000, the glass transition temperature was 131°C, the melt flow rate was 7.2 g/10 min, the lactone cyclizing rate was 94%, and the content of polymer gels was 5 pieces/100 g. The results are shown in Table 1.

Moreover, as for the obtained pellets of the lactone ring-contained polymer, when the kneading test was carried out on the same conditions as those used in Example 1, the content of polymer gels remained 5 pieces/100 g. The results are shown in Table 1.

The melt viscosity at 250°C of the obtained pellets of the lactone ring-containing polymer was measured to be 4,400 poise. Then, after the pellets of the lactone ring-containing polymer were left for 30 minutes in an oven at 280°C, the melt viscosity at 250°C of the pellets of the lactone ring-containing polymer was measured again to be 8,000 poise. From the melt viscosity measurements before and after heating, the increasing rate in viscosity (after heating/before heating) was 1.82. The results are shown in Table 1.

<Example 4>

A 30-L kettle type reactor equipped with a stirring device, a temperature sensor, a condenser, and a nitrogen introduction tube was charged with 8,000 g of methyl methacrylate (MMA), 2,000 g of methyl 2-hydroxymethylacrylate (MHMA; prepared in Synthetic Example 1), and 10,000 g of toluene, to which 12.0 g of dichlorotris(triphenylphosphine) ruthenium, 10.2 g of aluminum triisopropoxide, 4.7 g of 2,2'-dichloroacetophenone were added as polymerization initiators. The solution polymerization was carried out at about 105°C to 110°C under reflux, and further matured for 24 hours.

The obtained polymer solution was added in excessive hexane to precipitate a polymer. After the precipitate was washed with hexane and water, the precipitate was vacuum dried at 80°C under 1.33 hPa (1 mmHg) for three hours or longer, and the volatile components and the like were removed.

The obtained polymer was dissolved again in toluene to give the 50% solution. To this solution, a mixture of stearyl phosphate/distearyl phosphate (Phoslex A-18, available from Sakai Chemical Industry Co., Ltd.) was added at a rate of 0.001 g per 1 g of the polymer, and cyclocondensation reaction was carried out at about 90°C to 110°C for five hours under reflux. Then, in the same manner as described in Example 1, the obtained polymer solution was subjected to cyclocondensation reaction and devolatilizing in an extruder, and transparent pellets of a lactone ring-containing

polymer were obtained by extrusion.

As for the obtained lactone ring-containing polymer, the dynamic TG measurement was carried out, and 0.22% by mass in mass decrease was detected. Moreover, the characteristics of this lactone ring-containing polymer was as follows: the weight average molecular weight was 72,000, the glass transition temperature was 130°C, the melt flow rate was 32.5 g/10 min, the lactone cyclizing rate was 96%, and the content of polymer gels was 20 pieces/100 g. The results are shown in Table 1.

Moreover, as for the obtained pellets of the lactone ring-containing polymer,when the kneading test was carried out on the same conditions as those used in Example 1, the content of polymer gels remained 20 pieces/100 g. The results are shown in Table 1.

The melt viscosity at 250°C of the obtained pellets of the lactone ring-containing polymer was measured to be 1,700 poise. Then, after the pellets of the lactone ring-containing polymer were left for 30 minutes in an oven of 280°C, the melt viscosity at 250°C of the pellets of the lactone ring-containing polymer was measured again to be 2,500 poise. From the melt viscosity measurements before and after heating, the increasing rate in viscosity (after heating/before heating) was 1.47. The results are shown in Table 1.

<Comparative Example 1>

A 30-L kettle type reactor equipped with a stirring device, a temperature sensor, a condenser, and a nitrogen introduction tube was charged with 8,000 g of methyl methacrylate (MMA), 2,000 g of methyl 2-hydroxymethylacrylate (MHMA; prepared in Synthetic Example 2), 10,000 g of toluene, and 5.0 g of n-dodecyl mercaptan. The solution was heated to 100°C while passing nitrogen through the solution, and 10.0 g of t-butylperoxyisopropylcarbonate (Kayacarbon BIC-75, available from Kayaku Akzo Co., Ltd.) as a polymerization initiator was added when the reflux began, at which same time while dropping a solution containing 20.0 g of t-butylperoxyisopropylcarbonate and 100 g of toluene for two hours, the solution polymerization was carried out at about 95°C to 110°C under reflux, and further matured for four hours.

To the obtained polymer solution, 10 g of a mixture of stearyl phosphate/distearyl phosphate (Phoslex A-18, available from Sakai Chemical Industry Co., Ltd.) was added, and cyclocondensation reaction was carried out at about 90°C to 110°C for five hours under reflux. Then, in the same manner as described in Example 1, the obtained polymer solution was subjected to cyclocondensation reaction and devolatilizing in an extruder, and transparent pellets of the lactone ring-containing polymer were obtained by extrusion.

As for the obtained lactone ring-containing polymer, the dynamic TG measurement was carried out, and 0.24% by mass in mass decrease was detected. Moreover, the characteristics of this lactone ring-containing polymer were as follows: the weight average molecular weight was 133,000, the glass transition temperature was 130°C, the melt flow rate was 11.3 g/10 min, the lactone cyclizing rate was 96%, and the content of polymer gels was 130'pieces/100 g. The results are shown in Table 1.

Moreover, as for the obtained pellets of the lactone ring-containing polymer, when the kneading test was carried out on the same conditions as those used in Example 1, the content of polymer gels increased to 380 pieces/100 g. The results are shown in Table 1.

The melt viscosity at 250°C of the obtained pellets of the lactone ring-containing polymer was measured to be 2,400 poise. Then, after the pellets of the lactone ring-containing polymer were left for 30 minutes in an oven of 280°C, the melt viscosity at 250°C of the pellets of the lactone ring-containing polymer was measured again to be 103,400 poise. From the melt viscosity measurements before and after heating, the increasing rate in viscosity (after heating/before heating) was 43.08. The results are shown in Table 1.

<Comparative Example 2>

A 30-L kettle type reactor equipped with a stirring device, a temperature sensor, a condenser, and a nitrogen introduction tube was charged with 8,500 g of methyl methacrylate (MMA), 1,500 g of methyl 2-hydroxymethylacrylate (MHMA; prepared in Synthetic Example 2), 3,800 g of methyl isobutyl ketone (MIBK), and 950 g of methyl ethyl ketone (MEK). The initial concentration of monomers was made to be 68%, and 7.0 g of t-amyl-peroxy-3,5,5-trimethylhexanoate (Kayaester AN, available from Kayaku Akzo Co., Ltd.) as a polymerization initiator was added, at which same time while dropping a solution containing 7.0 g of t-amyl-peroxy-3,5,5-trimethylhexanoate, 280 g of MIBK, and 70 g of MEK for four hours, the solution polymerization was carried out at about 95°C to 110°C under reflux, and further matured for four hours. The mixed solvent (MIBK : MEK = 4 : 1) was dropped at a speed of 2,500 g/h from two hours later to four hours later of the beginning of the polymerization reaction, and at a speed of 1, 600 g/h from four hours later to seven hours later so that the concentration of polymer in the solution would be 45% or lower.

A mixture of stearyl phosphate/distearyl phosphate (Phoslex A-18, available from Sakai Chemical Industry Co., Ltd.) was added at a rate of 0.005 g per 1 g of the polymer component in the obtained polymer solution, and cyclocondensation reaction was carried out at about 80°C to 100°C for five hours under reflux while passing nitrogen through

the solution. Then, as for the obtained polymer solution, in the same manner as described in Example 1, cyclocondensation reaction and devolatilizing were carried out in an extruder, and transparent pellets of a lactone ring-containing polymer were obtained by extrusion.

As for the obtained lactone ring-containing polymer, the dynamic TG measurement was carried out, and 0.26% by mass in mass decrease was detected. Moreover, the characteristics of this lactone ring-containing polymer were as follows: the weight average molecular weight was 305,000, the glass transition temperature was 129°C, the melt flow rate was 0.5 g/10 min, the lactone cyclizing rate was 95%, and the content of polymer gels was 280 pieces/100 g. The results are shown in Table 1.

Moreover, as for the obtained pellets of the lactone ring-containing polymer, when the kneading test was carried out on the same conditions as those used in Example 1, the content of polymer gels increased to 830 pieces/100 g. The results are shown in Table 1.

TABLE 1

| | Weight average molecular weight | Glass transition temperature (°C) | Melt flow rate (g/10 min.) | Lactone cycling rate (%) | Content of polymer gels (pieces/100 g) | | Melt viscosity (poise) | | Increasing rate in viscosity (After heating/ Before heating) |
|---|---|---|---|---|---|---|---|---|---|
| | | | | | Before kneading test | After kneading test | Before heating | After heating | |
| Example 1 | 147,770 | 130 | 11.0 | 97 | 2 | 2 | 2,100 | 3,700 | 1.76 |
| Example 2 | 141,000 | 130 | 13.0 | 97 | 58 | 60 | 2,050 | 3,800 | 1.05 |
| Example 3 | 168,000 | 131 | 7.2 | 94 | 5 | 5 | 4,400 | 8,000 | 1.82 |
| Example | 72,000 | 130 | 32.5 | 96 | 20 | 20 | 1,700 | 2,500 | 1.47 |
| Comp. Ex. 1 | 133,000 | 130 | 11.3 | 96 | 130 | 380 | 2,400 | 103,400 | 43.08 |
| Comp. Ex. 2 | 305,000 | 129 | 0.5 | 95 | 280 | 830 | - | - | - |

As can be seen from Table 1, the lactone ring-containing polymers in Examples 1 and 2 were produced using a t-amyl type peroxide as the polymerization initiator, the lactone ring-containing polymer in Example 3 was produced using an initiator of azo series, and the lactone ring-containing polymer in Example 4 was produced using a living radical initiator, and all polymers have molecular weights in the prescribed range, so that these polymers show high lactone cyclizing rate, have high heat resistance, have melt flow rate suitable for forming and processing, have very few contents of polymer gels; therefore, these polymers are suitable particularly for optical applications.

In contrast, the lactone ring-containing polymer in Comparative Example 1 has a molecular weight in the prescribed range, but is produced using a t-butyl type peroxide as the polymerization initiator, and the lactone ring-containing polymer in Comparative Example 2 is produced using a t-amyl type peroxide, but has a molecular weight outside the prescribed range, so that these polymers show high lactone cyclizing rate and have high heat resistance, but the content of polymer gels is very high; therefore, these polymer are not suitable for optical applications.

Moreover, as for the lactone ring-containing polymer in Example 1 which was produced using high purity methyl 2-hydroxymethylacrylate prepared in Synthetic Example 1 and using a t-amyl type peroxide as the polymerization initiator, the content of polymer gels is very few, compared to the lactone ring-containing polymer in Example 2 which was produced using low purity methyl 2-hydroxymethylacrylate prepared in Synthetic Example 2 and using a t-amyl type peroxide as the polymerization initiator. This fact shows that in case of producing a lactone ring-containing polymer, when 2-hydroxymethylacrylic acid ester purified by the specific method is used as a monomer and a t-amyl type peroxide is used as the polymerization initiator, the generation of polymer gels can be very effectively suppressed.

Moreover, as can be seen from Table 1, in the lactone ring-containing polymers in Example 1 to 4, the increasing rate in viscosity after heating at 280°C for 30 minutes is 2.0 times or lower for the above reasons, and it is therefore considered that neither branching of the polymer molecular chain nor crosslinking between the molecules did not occur too much, even if being heated, so that gelation was suppressed.

In contrast, as for the lactone ring-containing polymer in Comparative Example 1, the melt viscosity rises rapidly by being heated probably because the polymer was produced using a t-butyl type peroxide as the polymerization initiator, and it is therefore considered that gelation occurred by branching of the polymer molecular chain and crosslinking between the molecules.

Thus, it is understood that in case of producing a lactone ring-containing polymer, not using t-butyl type peroxides, which have been used in the conventional producing method, but using polymerization initiators with the low ability of withdrawing hydrogen atoms from a polymer molecular chain, such as t-amyl type peroxides, azo initiators, and living radical initiators, the polymer is produced so that the molecular weight is in the prescribed range, which gives the lactone ring-containing polymer having few foreign matters and not easily causing gelation.

The lactone ring-containing polymer of the present invention contains very few foreign matters and does not easily cause gelation, in addition to being excellent in transparency and heat resistance as well as having the desired properties such as mechanical strength and forming and processing properties. Thus, the lactone ring-containing polymer of the present invention can widely be used in optical applications and the like, and will make a great contribution particularly to various fields related to optical materials.

## Claims

**1.** A lactone ring-containing polymer meeting a condition that:

(1) a content of polymer gels of 50 μm or greater in average particle diameter is lower than or equal to 100 pieces/100 g, and a weight average molecular weight of the polymer is 50,000 to 170,000.

**2.** The lactone ring-containing polymer according to claim 1, further meeting a condition that

(2) an increasing rate of viscosity after heated at 280°C for 30 minutes is 2.0 times or smaller.

**3.** The lactone ring-containing polymer according to claim 1 or 2, wherein the polymer has a lactone ring structure of formula (1):

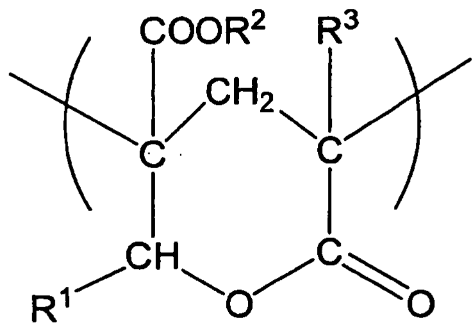

(1)

wherein $R^1$, $R^2$, and $R^3$ each independently means a hydrogen atom or an organic residue having 1 to 20 carbon atoms, in which the organic residue may contain an oxygen atom(s).

**4.** A forming material producible by heating and granulating the lactone ring-containing polymer according to any one of claims 1 to 3.

**5.** An optical component selected from a light guide material, an optical lens, and an optical film, all of which are formed from the forming material according to claim 4.

**Patentansprüche**

**1.** Lactonring-haltiges Polymer, welches eine Bedingung erfüllt, daß:

(1) ein Gehalt an Polymergelen von 50 μm oder größer im durchschnittlichen Teilchendurchmesser niedriger als oder gleich 100 Stücke/100 g und ein gewichtsmittleres Molekulargewicht des Polymers 50.000 bis 170.000 ist.

**2.** Lactonring-haltiges Polymer gemäß Anspruch 1, welches weiter eine Bedingung erfüllt, daß:

(2) eine Anstiegsrate der Viskosität nach Erwärmung bei 280°C für 30 Minuten 2,0-mal oder geringer ist.

**3.** Lactonring-haltiges Polymer gemäß Anspruch 1 oder 2, wobei das Polymer eine Lactonringstruktur der Formel (1) aufweist:

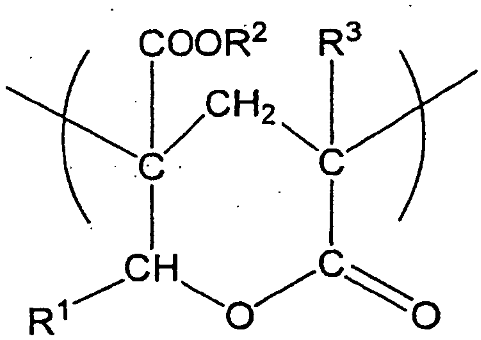

(1)

worin $R^1$, $R^2$ und $R^3$ jeweils unabhängig voneinander ein Wasserstoffatom oder einen organischen Rest mit 1 bis 20 Kohlenstoffatomen bedeuten, worin der organische Rest (ein) Sauerstoffatom(e) enthalten kann.

**4.** Formmaterial, erzeugbar durch Erwärmen und Granulieren des Lactonringhaltigen Polymers gemäß einem der Ansprüche 1 bis 3.

**5.** Optische Komponente, ausgewählt aus einem Wellenleitermaterial, einer optischen Linse und einem optischen

Film, welche sämtlich aus dem Formmaterial gemäß Anspruch 4 gebildet sind.

**Revendications**

1. Polymère contenant un noyau de lactone, satisfaisant à une condition telle que :

   (1) la teneur en gels de polymère ayant un diamètre moyen des particules de 50 μm ou plus est inférieure ou égale à 100 morceaux/100 g, et la masse moléculaire moyenne en poids du polymère est de 50 000 à 170 000.

2. Polymère contenant un noyau de lactone selon la revendication 1, satisfaisant en outre à une condition telle que :

   (2) le taux d'augmentation de la viscosité après chauffage à 280 °C pendant 30 minutes est de 2,0 fois ou moins.

3. Polymère contenant un noyau de lactone selon la revendication 1 ou 2, ledit polymère ayant une structure de noyau de lactone de formule (1) :

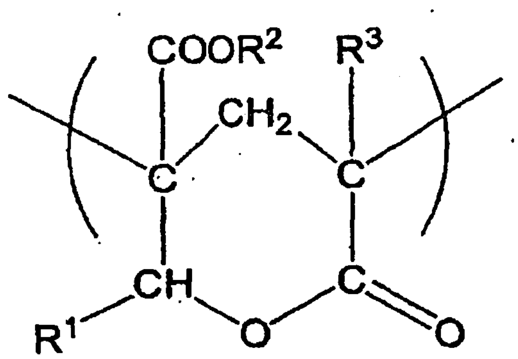

(1)

dans laquelle $R^1$, $R^2$ et $R^3$ signifient chacun indépendamment un atome d'hydrogène ou un résidu organique ayant de 1 à 20 atomes de carbone, ledit résidu organique pouvant contenir un ou plusieurs atomes d'oxygène.

4. Matériau de formage pouvant être produit par chauffage et granulation du polymère contenant un noyau de lactone selon l'une quelconque des revendications 1 à 3.

5. Composant optique choisi parmi un matériau guide de lumière, une lentille optique et un film optique, qui sont tous formés à partir du matériau de formage selon la revendication 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000230016 A
- JP 2005146084 A
- JP 9241323 A
- EP 1008606 A
- JP 2000302815 A
- JP 2004168882 A
- JP 2001151814 A
- JP 10053547 A
- JP 09067310 A
- JP 61254608 A
- JP 61261303 A